(19) Europäisches Patentamt
European Patent Office
Office européen des brevets

(11) Publication number: **0 196 849**
**A2**

(12) **EUROPEAN PATENT APPLICATION**

(21) Application number: 86302145.7

(22) Date of filing: 24.03.86

(51) Int. Cl.[4]: C 07 C 91/18
C 07 C 93/06, C 07 C 91/34
C 07 C 91/40, A 61 K 31/135
//C07C87/18, C07C93/08

(30) Priority: 27.03.85 GB 8507942

(43) Date of publication of application:
08.10.86 Bulletin 86/41

(84) Designated Contracting States:
BE CH DE FR GB IT LI LU NL SE

(71) Applicant: BEECHAM GROUP PLC
Beecham House Great West Road
Brentford Middlesex TW8 9BD(GB)

(72) Inventor: Smith, David Glynn
36 Shrewsbury Road
Redhill Surrey, RH1 6BH(GB)

(72) Inventor: White, Susan Mary
9 Longmeadow
Little Bookham Surrey, KT23 3AL(GB)

(74) Representative: Blake, John Henry Francis et al,
Beecham Pharmaceuticals Great Burgh Yew Tree
Bottom Road
Epsom, Surrey KT18 5XQ(GB)

# (54) Aminohydroxyalkylbenzene anti-obesity and/or anti-hyperglycaemic agents.

(57) A compound of formula (I):

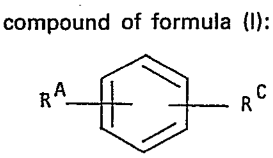

(I)

or a pharmaceutically acceptable salt thereof, wherein $R^A$ is a group of formula (a):

```
T
 \
  N-X²-          (a)
 /
T¹
```

wherein T represents a group of formula (b):

```
        OH
        |
Q-X¹-CH-CH₂      (b)
```

wherein Q is 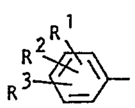, and where

$R^1$ is a hydrogen or halogen atom or a $-CF_3$, $OR^4$, $NHR^4$, $-NHCOR^4$, $-NHCONH_2$, $NHSO_2R^4$, $CH_2OR^4$ or $-CH_2SO_2R^4$ group:

$R^2$ is a hydrogen or halogen atom or an $-OR^4$ group;

$R^3$ is a hydrogen or halogen atome; and $R^4$ is hydrogen, $C_{1-6}$ alkyl or benzyl;

$X^1$ is a bond or $-OCH_2-$;

$X^2$ is a group of formula $-CR^5R^6-CH_2-A-$ wherein $R^5$ and $R^6$ are independently hydrogen or methyl; and

A is a bond, $-CH_2-$, or O;

$T^1$ represents a hydrogen atom or a group of formula (b) as defined above in respect of T, such that $T^1$ and T may be the same or different; $R^C$ represents a group (a) as defined above in respect of $R^A$, such that $R^C$ and $R^A$ may be the same or different; provided that when $R^A$ and $R^C$ each represent a group of formula:

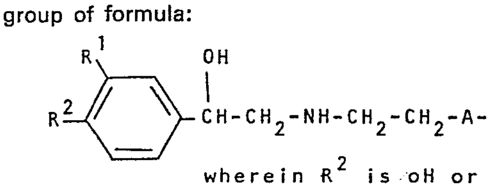

wherein $R^2$ is OH or $-O-$benzyl; then when A is a bond, $-CH_2-$ or $-O$, $R^1$ is not $-CH_2OH$ or $NHSO_2-C_{1-4}$ alkyl; processes for the preparation of such compounds and their use in medicine and agriculture.

EP 0 196 849 A2

## COMPOUNDS AND METHOD

This invention relates to a class of arylethanolamine derivatives, in particular to bridged bis(arylethanolamines), which have β-agonist activity; to processes for the preparation of such compounds and to their use in medicine and agriculture.

United States Patent No. 3,875,233 discloses compounds of a general formula:

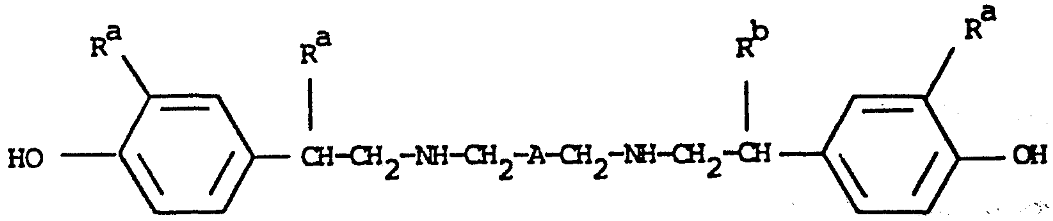

wherein $R^a$ is hydroxyl, hydroxymethyl or

$-NH-SO_2-R^c$

wherein $R^c$ is alkyl of 1 to 4 carbon atoms:
$R^b$ is hydroxymethyl when $R^a$ is hydroxyl: or hydroxyl when $R^a$ is other than hydroxyl; and
A is
i. when $R^a$ is hydroxyl.

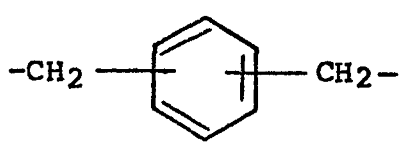

wherein the ring substituents are ortho or meta to each other: or

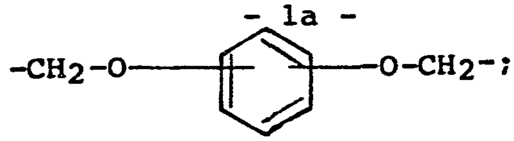

ii. when $R^a$ is hydroxymethyl.

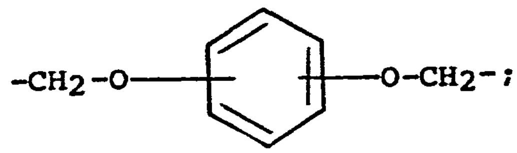 or

iii. when $R^a$ is $-NH-SO_2-R^c$ wherein $R^c$ is as defined above,

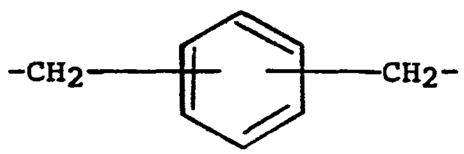 or

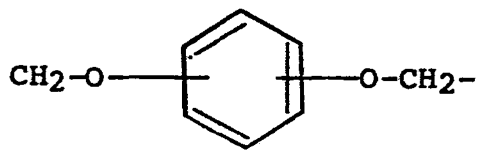 or

iv. when $R^a$ is $-NH-SO_2-R^c$ wherein $R^c$ is as defined above,

$-(CH_2)_n-$ wherein n is 0 or an integer from 1 to 8.

The compounds of USP 3875233 are described as being useful as bronchospasmolytic agents in animals.

French Demande Published Application No. 2,147,188 discloses compounds of general formula:

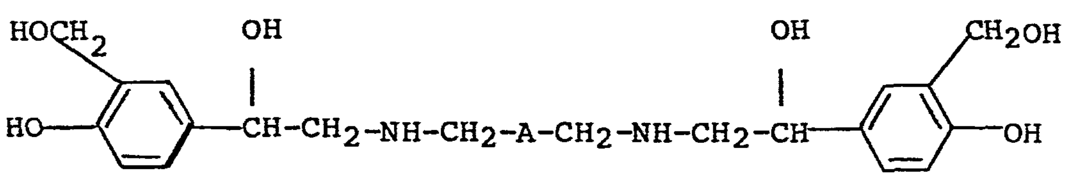

wherein A represents $-(CH_2)_n$ or $-(CH_2)_m$ 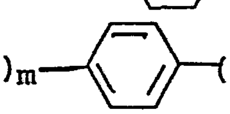 $(CH_2)_m-$ wherein m = 1 or 2 and n = 0-8.

The compounds of FR 2147188 are described as being useful bronchospasmolytic agents.

It has now surprisingly been discovered that certain bridged bis(arylethanolamines) have good anti-obesity and/or anti-hyperglycaemic activity. These compounds are also useful as growth promoters for animals.

Accordingly, the present invention provides a method for treating obesity and/or hyperglycaemia in human or non-human animals which method comprises administering an effective non-toxic amount of a compound of formula (A):

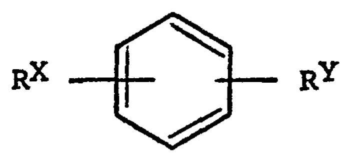

(A)

or a pharmaceutically acceptable salt thereof, wherein $R^X$ is a group of formula (a):

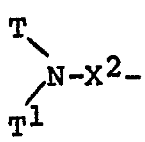

(a)

wherein T represents a group of formula (b):

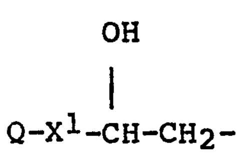

(b)

wherein Q is 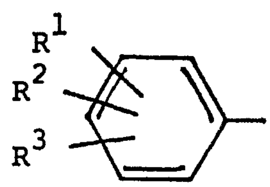 , and wherein

$R^1$ is a hydrogen or halogen atom or a $-CF_3$, $OR^4$, $NHR^4$, $-NHCOR^4$, $-NHCONH_2$, $NHSO_2R^4$, $CH_2OR^4$ or $-CH_2SO_2R^4$ group;

$R^2$ is a hydrogen or halogen atom or an $-OR^4$ group;

$R^3$ is a hydrogen or halogen atom; and $R^4$ is hydrogen, $C_{1-6}$ alkyl or benzyl;

$X^1$ is a bond or $-OCH_2-$;

$X^2$ is a group of formula $-CR^5R^6-CH_2-A-$

wherein $R^5$ and $R^6$ are independently hydrogen or $C_{1-6}$ alkyl; and

A is a bond, $-CH_2-$, or O;

$T^1$ represents a hydrogen atom or a group of formula (b) as defined above in respect of T, such that $T^1$ and T may be the same or different; $R^Y$ represents a group of formula (a), as defined above in respect of $R^X$, such that $R^Y$ and $R^X$ may be the same or different; to an obese and/or hyperglycaemic human or non-human animal.

Preferably, $R^X$ and $R^Y$ are para-substituents with respect to each other.

In a further aspect the present invention also provides the use of the hereinbefore defined compound of formula (A) for the manufacture of a medicament for the treatment of obesity and/or hyperglycaemia.

In a further aspect the present invention also provides a compound of formula (I):

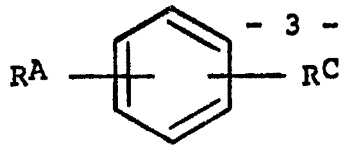

(I)

or a pharmaceutically acceptable salt thereof, wherein $R^A$ is a group of formula (a):

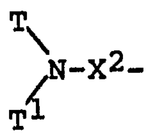

(a)

wherein T represents a group of formula (b):

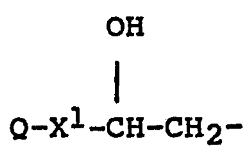

(b)

wherein Q is 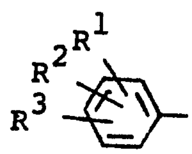 , and wherein

$R^1$ is a hydrogen or halogen atom or a $-CF_3$, $OR^4$, $NHR^4$, $-NHCOR^4$, $-NHCONH_2$, $NHSO_2R^4$, $CH_2OR^4$ or $-CH_2SO_2R^4$ group;

$R^2$ is a hydrogen or halogen atom or an $-OR^4$ group;

$R^3$ is a hydrogen or halogen atom; and $R^4$ is hydrogen, $C_{1-6}$ alkyl or benzyl;

$X^1$ is a bond or $-OCH_2-$;

$X^2$ is a group of formula $-CR^5R^6-CH_2-A-$

wherein $R^5$ and $R^6$ are independently hydrogen or methyl; and

A is a bond, $-CH_2-$, or O;

$T^1$ represents a hydrogen atom or a group of formula (b) as defined above in respect of T, such that $T^1$ and T may be the same or different; $R^C$ represents a group (a) as defined above in respect of $R^A$, such that $R^C$ and $R^A$ may be the same or different; provided that when $R^A$ and $R^C$ each represent a group of formula:

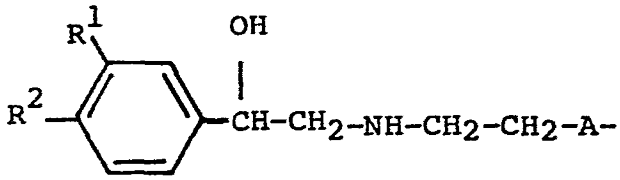 wherein $R^2$ is OH or -O-benzyl; then when A is a bond, $-CH_2-$ or -O, $R^1$ is not $-CH_2OH$ or $NHSO_2-C_{1-4}$ alkyl.

Preferably $R^A$ and $R^B$ are para-substituents with respect to each other.

Suitably, $T^1$ represents hydrogen.

Aptly, $R^1$ is hydrogen, chlorine, $-CF_3$, hydroxyl, $NH_2$, $NHCOC_{1-6}$alkyl, $NHCONH_2$, $NHSO_2C_{1-6}$alkyl, $CH_2OH$ or $CH_2SO_2C_{1-6}$alkyl.

Favourably, $R^1$ represents hydrogen, chlorine, $-CF_3$, hydroxyl, $NH_2$, $NHCOC_{1-6}$ alkyl, $NHCONH_2$ or $CH_2SO_2C_{1-6}$ alkyl.

Preferably, $R^1$ represents hydrogen, chlorine, hydroxy or amino.

A suitable sub-group of the compounds of formula (I) is provided by those compounds of formula (II):

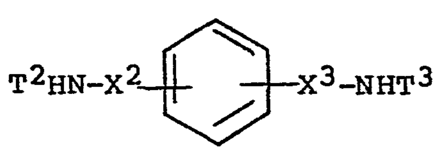 (II)

or a pharmaceutically acceptable salt thereof, wherein $T^2$ and $T^3$ each independently represent a group T as defined in relation to formula (I), and $X^2$ and $X^3$ each independently represent a group of formula $-CR^5R^6-CH_2A$ as defined in relation to formula (I).

Suitably, $X^2 = X^3$

A further suitable sub-group of the compounds of formula (I) are those compounds of formula (III):

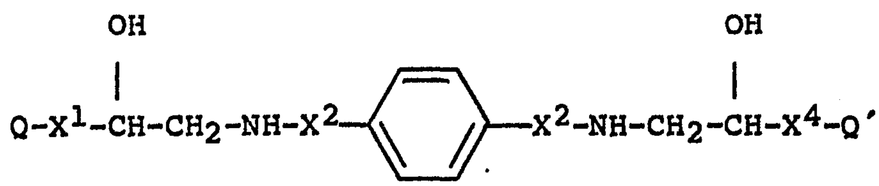

(III)

or a pharmaceutically acceptable salt thereof, wherein $X^2$ is as defined in relation to formula (I), Q and Q′ each independently represent a group

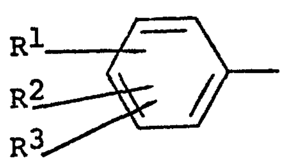

as defined in relation to formula (I); and $X^1$ and $X^4$ each independently represent a bond or $-OCH_2-$ .

Favourably, $X^2$ is a group of formula 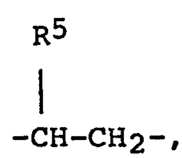, wherein $R^5$ is as defined in relation to formula (I).

$R^5$
|
Favourably, $X^2$ is a group of formula $-CH-CH_2-O-$ wherein $R^5$ is as defined in relation to formula (I).

Suitably, Q and Q′ are each independently represented by a group

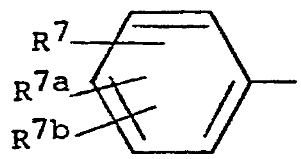

wherein $R^7$ is a hydrogen, chlorine or bromine atom or a hydroxyl or amino group; $R^{7a}$ and $R^{7b}$ each independently represent a hydrogen or halogen atom.

Favourably, $R^7$ is a hydrogen atom.

Favourably, $R^7$ is a chlorine atom, preferably attached to the benzene ring in the meta-position relative to the point of attachment of the rest of the molecule.

Favourably, $R^7$ is a hydroxyl group, preferably attached to the benzene ring in the para-position relative to the point of attachment of the rest of the molecule.

Favourably, $R^7$ represents an amino group, preferably attached to the benzene ring in the meta-position relative to the point of attachment of the rest of the molecule.

Favourably, $R^{7a}$ and $R^{7b}$ each independently represent a hydrogen or chlorine atom.

Preferably, Q and Q′ each independently represent phenyl, 3-chlorophenyl, 3,4-dichlorophenyl, 3,5-dichloro-4-amino phenyl or 4-hydroxyphenyl.

A preferred group of compounds of the present invention which falls within the sub-group of compounds of formula (III) are those of formula (IV):

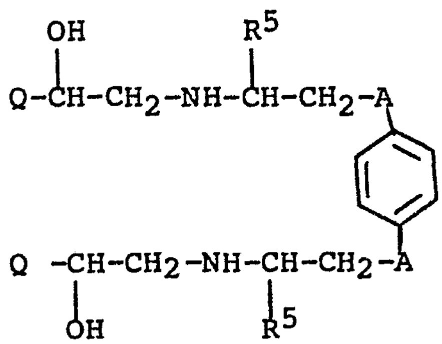

(IV)

or pharmaceutically acceptable salts thereof, wherein Q, A and $R^5$ are as defined in relation to formula (I). A further preferred group of compounds of formula (I) which falls within the sub-group of compounds of formula (III) are those of formula (V);

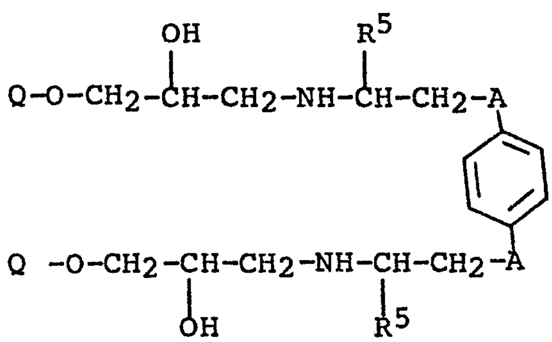

(V)

or pharmaceutically acceptable salts thereof, wherein Q, A, and $R^5$ are as defined in relation to formula (I).

In a preferred aspect the present invention provides a compound selected from the list consisting of:

(R,R,R,R)-α,α'-[1,4-phenylenebis[(1-methyl-2,1-ethanediyl) imino]methylene]bis[benzenemethanol];

(R,R,R,R)-α,α'-[1,4-phenylene bis[(1-methyl-2,1-ethanediyl)imino]methylene]bis[3-chlorobenzenemethanol]

(R,R)-α-α'-[1,4-phenylenedioxybis [(2,1-ethanediyl)-imino] methylene] bis [benzenemethanol];

(R,R)-α,α'-[1,4-phenylenebis [(2,1-ethanediyl) imino]-methylene]bis[benzenemethanol];

(R,R)-α,α1-[1,4-phenylenedioxybis[(2,1-ethanediyl)-imino]methylene]bis[3-chlorobenzene methanol];

(R,R)-α,α'-[1,4-phenylenebis[(2,1-ethanediyl]imino]-methylene]bis[3-chlorobenzene methanol];

α,α'-[1,4-phenylenedioxybis [(1-methyl-2,1-ethanediyl) imino]methylene]bis[(R)-3-chlorobenzenemethanol];

(R,R)-α,α'-[1,2-phenylenebis[(2,1-ethanediyl)imino] methylene] bis [3-chlorobenzenemethanol];

(R,R,R,R)-α,α'-[1,4-phenylenebis [(1-methyl-2,1-ethanediyl)imino] methylene] bis [3,4-dichlorobenzenemethanol];

(R,R,R,R)-1,1'-[1,4-phenylenebis [(1-methyl-2,1-ethanediyl)imino]] bis [3-phenoxypropan-2-ol];

1,1'-[1,4-phenylenebis [((R)-1-methyl-2,1-ethanediyl) imino]]bis [3-phenoxypropan-2-(S)-ol];

(R,R,R)-3,4-dichloro-α-[[[2-[4-[2-[[2-(3-chlorophenyl)-2-hydroxyethyl] amino] ethyloxy] phenyl]-1-methylethyl] amino] methyl] benzenemethanol;

(R,R,R,R)-α,α$^1$-[1,4-phenylenebis [(1-methyl-2,1-ethanediyl)imino]methylene] bis [4-hydroxybenzene-methanol];

α,α$^1$-[1,4-phenylenebis[((R)-1-methyl-2,1-ethanediyl) imino]methylene] bis [(4-amino-3,5-dichloro)benzene-methanol];

(R,R,R,R)-3-chloro-α-[[[2-[4-[2-[[2-(4-hydroxyphenyl)-2-hydroxyethyl]amino]propyl]phenyl]-1-methylethyl] amino]methyl]benzenemethanol;

3-chloro-α-(R)-[[[2-[4-[2-(R)-[(3-phenoxy-2-(S)-hydroxy propyl)amino]propyl]phenyl]-1-(R)-methylethyl]-amino]me thyl]benzenemethanol;

3-chloro-α-(R)-[[[2-[4-[2-(R)-[bis(3-phenoxy-2-(S)-hydr oxypropyl)amino]propyl]phenyl]-1-(R)-methylethyl]-amino ]methyl]benzenemethanol;

4-amino-3,5-dichloro-α-[[[2-[4-[2-(R)-[[2-(R)-(4-hydroxyphenyl)-2-hydroxyethyl]amino]propyl]phenyl]-1-(R)-methylethyl]amino]methyl]benzenemethanol;

4-hydroxy-α-(R)-[[[2-[4-[2-(R)-[(3-phenoxy-2-(S)-hydroxypropyl)amino]propyl]phenyl]-1-(R)-methylethyl]-amino]methyl]benzenemethanol;

4-hydroxy-α-(R)-[[[2-[4-[2-(R)-[bis(3-phenoxy-2-(S)-hydroxypropyl)amino]propyl]phenyl]-1-(R)-methylethyl]-amino]methyl]benzenemethanol; and

4-amino-3,5-dichloro-α-[[[2-[4-[2-(R)-[[2-(R)-(3-chlorophenyl)-2-hydroxyethyl]amino]propyl]phenyl]-1-(R)-methylethyl]amino]methyl]benzenemethanol; or a pharmaceutically acceptable salt thereof.

Suitable salts of the compounds of the invention include the acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicylic acid or acetylsalicylic acid.

When $R^5 \neq R^6$ and $T^1 \neq$ hydrogen, the compounds of formula (A) and of formula (I) have six asymmetric carbon atoms, marked 1* to 6* in the formula IA below:

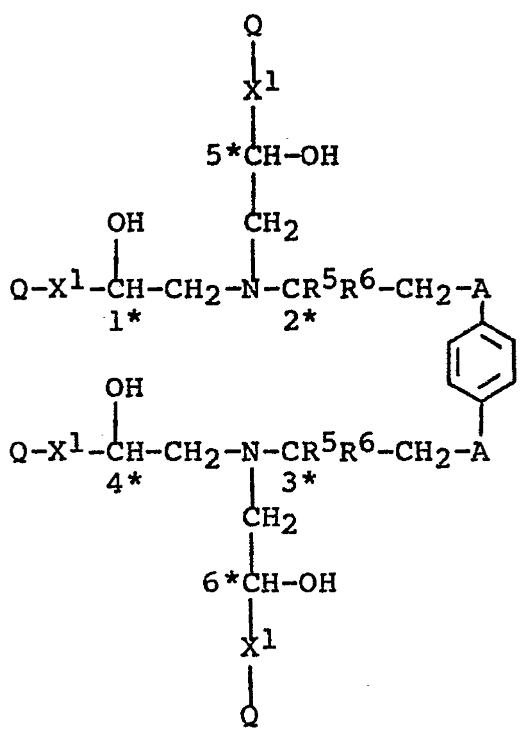

(1A)

These compounds may, therefore, exist in sixty four stereoisomeric forms. The present invention encompasses all stereoisomers of the compounds of the invention of formula (A) and formula (I) whether free from other stereoisomers or admixed with other stereoisomers in any proportion and this includes, for instance, racemic mixtures of enantiomers.

Suitably, when $X^1$ represents a bond the 1*, 4*, 5* and 6* asymmetric carbons have the R- configuration.

Suitably, when $X^1$ represents $-O-CH_2-$ the 1*, 4*, 5* and 6* asymmetric carbons have the S- configuration.

Suitably, when $R^5 \neq R^6$ the 2* and 3* asymmetric carbons have the R- configuration.

When $X^1$ is a bond, $T^1$ is hydrogen and $R^5=R^6$ a favoured enantiomer is the R,R enantiomer.

When $X^1$ is a bond, $T^1$ is hydrogen and $R^5 \neq R^6$ a favoured enantiomer is the RRRR enantiomer.

When $X^1$ is $-OCH_2-$, $T^1$ is hydrogen and $R^5=R^6$ a favoured enantiomer is the S,S enantiomer.

When $X^1$ is $-OCH_2-$, $T^1$ is hydrogen and $R^5 \neq R^6$ a favoured enantiomer is the SRRS enantiomer.

The absolute configuration of any compound of formula (A) or formula (I) may be determined by conventional X-ray crystallographic techniques.

The present invention also provides a process for the preparation of a compound of formula (I) from a compound of formula (VI):

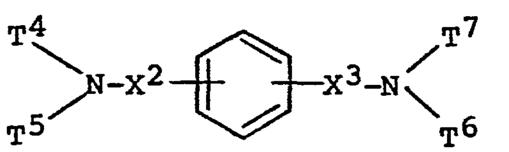

(VI)

wherein $X^2$ and $X^3$ are as defined in relation to formula (II), $T^4$ and $T^7$ each independently represents a hydrogen atom, a nitrogen protecting group or a moiety of formula (b), (c) or (d):

(b) $Q\text{-}X^1\text{-}\overset{\overset{\large OH}{|}}{C}H\text{-}CH_2\text{-}$;

(c) $Q\text{-}X^1\text{-}\overset{\overset{\large OH}{|}}{C}H\text{-}CO\text{-}$;

(d) $Q\text{-}X^1\text{-}CO\text{-}CH_2\text{-}$;

and $T^5$ and $T^6$ each independently represent a hydrogen atom, a nitrogen protecting group or a moiety (b) or $T^4$ together with $T^5$; or $T^6$ together with $T^7$ independently represent a moiety of formula (e)

(e) $Q\text{-}X^1\text{-}CO\text{-}CH\text{=}$;

wherein Q and $X^1$ are as defined in relation to formula (I) and providing that either $-X^2-NT^4T^5$ or $-X^3-NT^6T^7$ is not a moiety (a) as defined above, which process comprises carrying out the required number of the following steps:

(i) reducing a moiety (c), (d) or (e) in the compound of formula (VI);

(ii) protecting any -NH- moiety in the compound of formula (VI);

(iii) converting a compound of formula (VI), wherein $T^4$ or $T^7$ represents hydrogen, into another compound of formula (VI) or a compound of formula (I), by reaction with a compound of formula (VIA):

$$Q''\text{-}X^1\text{-}T^8 \qquad \text{(VIA)}$$

wherein $X^1$ is as defined above, Q'' represents a group Q such that Q and Q'' may be the same or different and $T^8$ represents a moiety (f), (g), (h) or (j):

(f) 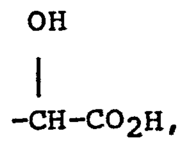

(g) 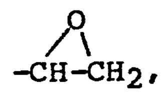

(h) $-CO-CH_2-R^x$,

(j) 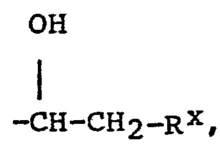

wherein $R^x$ is a leaving group.

(iv) converting a compound of formula (VI) wherein $-NT^4T^5$ or $-NT^6T^7$ represents $-NH_2$ into another compound of formula (VI), by reaction with a compound of formula (VIA) wherein $Q''$ and $X^1$ are as defined above and $T^8$ represents a moiety of formula (k)

(k) $-CO-CHO$

(v) removing any protecting group;

(vi) separating any required product;

(vii) repeating any step (i), (ii), (iii), (iv), (v) or (vi); and if required thereafter forming a pharmaceutically acceptable salt of the compound of formula (I).

In one aspect of the process of the present invention there is provided a process for the preparation of a compound of formula (I), from a compound of formula (VII):

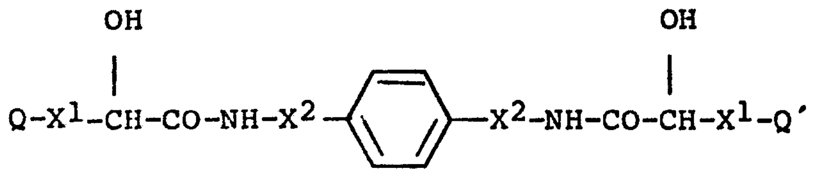 (VII)

or a pharmaceutically acceptable salt thereof, wherein Q, $X^1$ and $X^2$ are as defined in relation to formula (I) and Q′ is as defined in relation to formula (III) which process comprises:

(A) for preparing compounds of formula (I) wherein $T^1$ represents a hydrogen atom; reducing the compound of formula (VII);

or (B) for preparing compounds of formula (I) wherein $T^1$ represents a hydrogen atom or a group of the, hereinbefore defined, formula (b); carrying out the required number of the above defined steps (i), (ii), (iii), (v), (vi) and where appropriate (vii); and if necessary thereafter forming a pharmaceutically acceptable salt of the compound of formula (I).

The abovementioned reduction of the compound of formula (VII), and the reduction of any moiety (c) in process step (i), may conveniently be carried out using a complex metal hydride, for example lithium aluminium hydride, or borane methyl sulphide complex in an aprotic solvent, for example tetrahydrofuran or diethylether or a mixture of such solvents, at any convenient temperature, for example the reflux temperature of tetrahydrofuran or diethyl ether or a mixture of such solvents.

The desired product in the abovementioned reactions may be obtained from the reaction mixture by any conventional means such as filtration and evaporation. The initially obtained product may be purified by conventional means, for example by chromatography, crystallization or the like.

The reduction of the compound of formula (VII) to the compound of formula (I) proceeds via an intermediate compound of formula (VIIB):

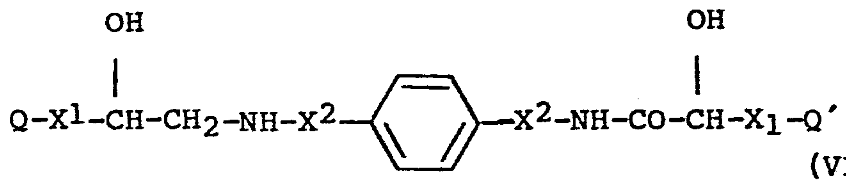

(VIIB)

wherein Q, $X^1$ and $X^2$ are as defined in relation to formula (VI) and Q′ is as defined in relation to formula (III).

In the reduction of the said compounds of formula (VII) to the compounds of formula (I) the compounds of formula (VIIB) are normally not isolated. However we have found that certain of the compounds of formula (VIIB) are active in their own right.

Accordingly, in a further aspect the present invention provides a compound of the hereinbefore defined formula (VIIB).

In a further aspect the present invention also provides a compound of the hereinbefore defined formula (VIIB) for use in the treatment of obese and/or hyperglycaemic humans or non-human animals.

The present invention also encompasses a method of treatment of obesity and/or hyperglycaemia, which method comprises administering an effective, non-toxic amount of a compound of formula (VIIB) to an obese and/or hyperglycaemic human or non-human animal.

The compounds of formula (VII) are suitably prepared from a compound of formula (VIII):

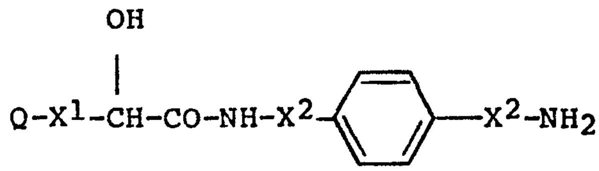

(VIII)

or a salt thereof, wherein Q, $X^1$ and $X^2$ are as defined in relation to formula (VII), by reaction with a compound of formula (IX):

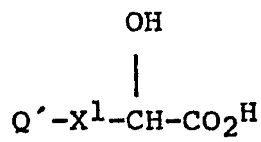

(IX)

wherein Q and $X^1$ are as defined in relation to formula (VII) and Q′ is as defined in relation to formula (III).

The compounds of formula (VIII) are suitably prepared by reaction of the hereinbefore defined compound of formula (IX) with a compound of formula (X):

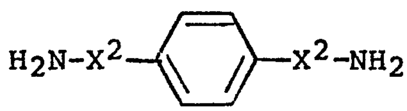

(X)

wherein $X^2$ and is as defined in relation to formula (VII).

The reaction between compounds (VIII) and (IX) and between compounds (IX) and (X) may take place under standard peptide formation reaction conditions, for example in the presence of dicyclohexylcarbodiimide, 1-hydroxybenzotriazole and dimethylformamide. Similar conditions may also be employed for the reaction between compound (VI) and (VIA) in process step (iii) wherein $T^8$ in compound (VIA) represents a moiety of formula (f).

In a preferred form of the process to prepare a compound of formula (VI) the compound of formula (VIII) is not isolated; the compound of formula (X) being treated <u>in-situ</u> with a compound of formula (IX); and in a particularly preferred form of the process the compound of formula (X) is treated with two molar equivalents of a compound of formula (IX).

The compounds of formula (X) may be prepared from a compound of formula (XA):

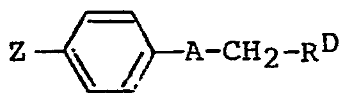

(XA)

wherein A is as defined in relation to formula (I); $R^D$ is -CN or $-COCH_3$; Z is another group $-A-CH_2-R^D$, as herein defined, or a group $-A-CH_2-CR^5R^6-NH_2$ wherein $R^5$ and $R^6$ are independently H or $CH_3$; by one or more of the following steps;

a) converting any group -CN to a group $-CH_2-NH_2$;

b) converting any group $-CO.CH_3$ to a group $-CH(CH_3)NH_2$;

c) converting any group $-CO.CH_3$ to a group $-C(CH_3)_2NH_2$;

d) if necessary protecting any amino group;

e) if necessary removing any amino protecting group.

The conversion of any group -CN to any group $-CH_2-NH_2$ or of any group $-CO.CH_3$ to a group $-CH(CH_3)NH_2$ or a group $-C(CH_3)_2NH_2$ may be carried out by methods conventional in the art.

In a further aspect the compounds of formula (I) may also be prepared from a compound of formula (XI):

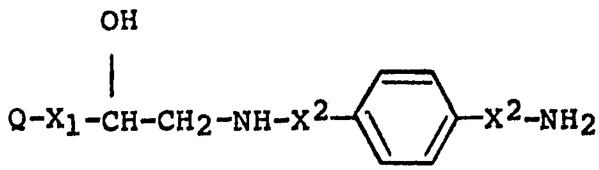

(XI)

wherein Q, $X^1$ and $X^2$ are as defined in relation to formula (I):

by either:

(A) for compounds of formula (I) wherein $T^1$ represents a hydrogen atom; reacting the compound of formula (XI) with a compound of formula (XII):

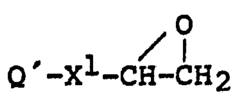

(XII)

wherein Q and $X^1$ are as hereinbefore defined and Q′ is as defined in relation to formula (III); or

(B) for compounds of formula (I) wherein $T^1$ represents a hydrogen atom or a group of the, hereinbefore defined, formula (b), carrying out the required number of the above defined steps (i), (ii), (iii), (iv), (v), (vi) or (vii);

and if necessary thereafter forming a pharmaceutically acceptable salt of the compound of formula (I).

The reaction between compounds (XI) and XII) and the reaction between compounds (VI) and (VIA) (in process step (iii) wherein $T^8$ in compound (VIA) represents a moiety of formula (g)) are normally carried out in a protic solvent such as a lower alkanol, preferably ethanol.

The compound of formula (XI) may be prepared by reacting the hereinbefore defined compound of formula (X) with the hereinbefore defined compound of formula (XII) under similar conditions.

In one form of the process to provide a compound of the invention of formula (I) wherein $T^1$ represents hydrogen, the compound of formula (XI) is not isolated; the compound of formula (XI) being treated in-situ with a compound of formula (XII); and in a particularly preferred form of the process for compounds wherein Q = Q′ the compound of formula (X) is reacted with two molar equivalents of a compound of formula (XII). Similarly, in the preparation of compounds of formula (I) wherein one or both variables $T^1$ represent a group of the hereinbefore defined formula (b), the compound of formula (X) may be reacted with 3 or 4 molar equivalents of (XII), respectively.

The compounds of formula (XI) may also be prepared from a compound of formula (XIA):

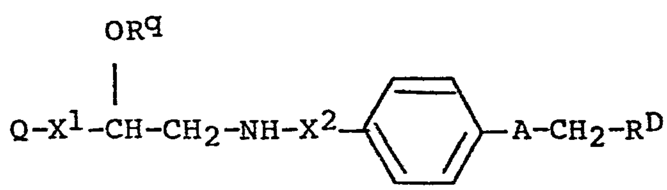

(XIA)

wherein Q,$X^1$,$X^2$, and A are as defined in relation to formula (I) and $R^D$ is as defined in relation to formula (XA) and $R^q$ is hydrogen or a protecting group; by using one or more of the steps used to prepare the hereinbefore defined compound of formula (X) from the hereinbefore defined compound of formula (XA) and if necessary thereafter removing any protecting group $R^q$.

The hydroxyl protecting group $R^q$ may be any hydroxyl protecting group used conventionally in the art.

The compounds of formula (XIA) may be prepared from compounds of formula ($X^A$) by processes analogous to those hereinbefore defined.

In one particular aspect of the process of the invention the compounds of the formula (I) may be prepared from a compound of formula (XIII):

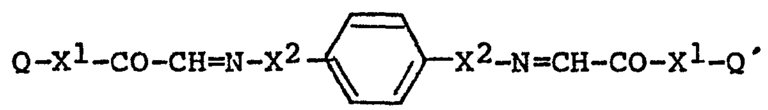 (XIII)

wherein Q,$X^1$ and $X^2$ are as defined in relation to formula (I) and Q′ is as defined in relation to formula (III), by either:

(A) for compounds of formula (I) wherein $T^1$ represents a hydrogen atom, reducing the compound of formula (XIII);

or (B) for compounds of formula (I) wherein $T^1$ represents a hydrogen atom or a group of the hereinbefore defined formula (b), by reducing the compound of formula (XIII) and thereafter carrying out the required number of the above defined steps (i), (ii), (iii), (iv), (v), (vi) or (vii); and if necessary thereafter forming a pharmaceutically acceptable salt of the compound of formula (I).

The reduction of the compound of formula (XIII), or the moiety (e) (in the above defined process step (i)) to the compound of formula (I), may proceed by way of a number of partially reduced intermediates, for example wherein any group -CO-CH=N- has been partially reduced to a group -CHOH-CH=N- or -CO-$CH_2$-NH-; in the abovementioned reaction the intermediate compounds are generally not isolated, they are normally reduced <u>in-situ</u>.

The reduction of the compounds of the formula (XIII) and of any moiety (e) and the above mentioned partially reduced intermediates may be effected using a complex hydride such as sodium borohydride or by catalytic hydrogenation.

The sodium borohydride reduction is generally carried out in a lower alkanolic solvent, for example methanol. An approximately ambient temperature may be employed, for example 20° to 30°C.

The desired compound may be obtained from the reaction mixture by evaporation, extraction into a suitable solvent such as ethyl acetate and evaporation. The initially obtained product may be purified by conventional means, for example by chromatography, crystallisation or the like.

In the catalytic hydrogenation suitable catalysts include noble metal catalysts such as palladium, for example palladium on charcoal or the like such as platinum for example as platinum oxide or Raney Nickel.

If Raney Nickel is used as catalyst hydrogen may be employed at ambient to medium pressures, for example a pressure of between 1 and 4 atmospheres. The reaction may be carried out at any convenient non-extreme temperature but it is generally most suitable to use an ambient or a slightly super ambient temperature, preferably an ambient temperature. The hydrogenation may be carried out in a conventional hydrogenation solvent such as a lower alkanol, for example ethanol.

The desired compound may be isolated from the reaction mixture by evaporation of the filtered solution. The initially obtained product may be purified by

conventional means, for example by chromatography, crystallistion or the like.

The compound of formula (XIII) may be prepared by reacting a compound of formula (XIV):

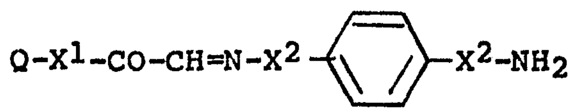

(XIV)

wherein Q, $X^1$ and $X^2$ are as defined in relation to formula (I) with a compound of formula (XV):

$$Q'-X_1-CO-CHO \qquad (XV)$$

or a hydrate of hemi-acetal thereof, wherein Q and $X^1$ are as defined in relation to formula (I) and Q′ is as defined in relation to formula (III).

The condensation reaction is generally carried out under conditions that result in the removal of water formed during the reacton. A convenient method is to remove azeotropically the water from a refluxing benzene solution using a Dean and Stark apparatus. Similar reaction conditions may also be employed in the reaction between compounds of formula (VI) and (VIA) in the above defined process step (iv).

The compound of formula (XIV) may be prepared by reacting the hereinbefore defined compound of formula (X), with the hereinbefore defined compound of formula (XV). The conditions of the reaction are suitably those described for the reaction between compounds of formula (XIV) and (XV).

In a preferred form of the process to prepare a compound of formula (XIII), the compound of formula (XIV) is not isolated; the compound of formula (XIV)

being reacted in-situ with a compound of formula (XV); and in a particularly preferred form of the process for compounds wherein $Q = Q'$ a compound of the hereinbefore defined formula (X) is reacted with at least two molar equivalents of a compound of formula (XV).

In a preferred form of the process to prepare a compound of formula (I) wherein $T^1$ represents hydrogen, the compound of formula (XIII) is not isolated; the compound of formula (XIII) being reduced in-situ as hereinbefore described.

In a particularly preferred form of the process to prepare a compound of formula (I) wherein $T^1$ represents hydrogen, the compounds of formula (XIV) and (XIII) are not isolated; the compound of formula (XIV) being reacted in-situ with a compound of formula (XV); the resulting compound of formula (XIII) being reduced in-situ as hereinbefore described to the compound of formula (I).

In a further aspect of the process of the invention, the compounds of formula (I) may be prepared from a compound of formula (XVI):

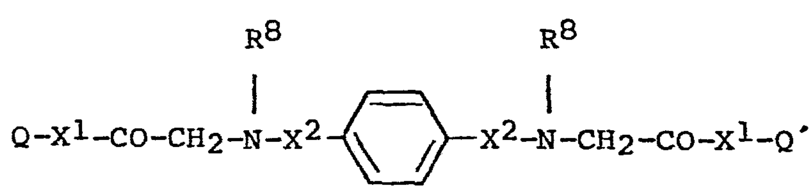

(XVI)

wherein $Q, X^1$ and $X^2$ are as defined in relation to formula (I), $Q'$ is as defined in relation to formula (III) and $R^8$ is a hydrogen atom or a benzyl group by either:

(A) for compounds of formula (I) wherein $T^1$ represents a hydrogen atom, reducing the compound of formula (XVI);

or (B) for compounds of formula (I) wherein $T^1$ represents a hydrogen atom or a group of the, hereinbefore defined, formula (b); carrying out process steps (i) and thereafter the required number of the above defined process steps (i), (ii), (iii), (iv), (v), (vi) or (viii);

and if necessary thereafter forming a pharmaceutically acceptable salt of the compound of formula (I).

The reduction of the compound of formula (XVI) to the compound of formula (I), wherein $T^1$ represents hydrogen, proceeds via an intermediate compound of formula (XVIA):

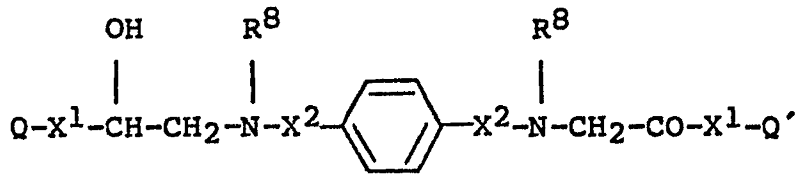
(XVIA)

wherein Q, Q′, $X^1$, $R^8$ and $X^2$ are as defined in relation to formula (XVI).

The compounds of formula (XVIA) are generally not isolated, they are normally reduced <u>in-situ</u> to the said compounds of formula (I).

The reduction of the compounds of formula (XVI) and (XVIA) and of any moiety of formula (d) (in the above defined process step (i)), may be effected by a complex metal hydride or by catalytic hydrogenation.

The complex metal hydride reduction may be carried out under conditions described for the reduction of a compound of formula (XIII). The hydrogenation may be carried out under conditions described for the hydrogenation of a compound of formula (XIII).

The compound of formula (XVI) may be prepared by reacting a compound of formula (XVII):

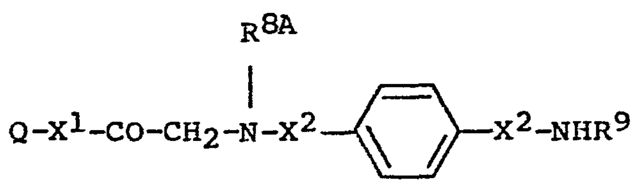

(XVII)

wherein Q, $X^1$ and $X^2$ and are as defined in relation to formula (I), $R^{8A}$ is a benzyl group and $R^9$ is H or a benzyl group, with a compound of formula (XVIII):

$$Q'-X^1-CO-CH_2-R^{10} \qquad (XVIII)$$

wherein Q and $X^1$ are as defined in relation to formula (I), Q′ is as defined in relation to formula (III) and $R^{10}$ is a leaving group; and thereafter if necessary converting any group $R^{8A}$ to a hydrogen atom or any group $R^9$ to another group $R^9$.

Suitably, $R^9$ is a benzyl group

Suitably, $R^{10}$ is a bromine atom or a tosylate group.

This reaction and the reaction between compounds (VI) and (VIA) in the above defined process step (iii) (wherein $T^8$ in compound (VIIA) represents a moiety (h) wherein $R^x$ represents a group $R^{10}$) may be carried out in a solvent such as acetonitrile or butanone at an elevated temperature, for example under reflux. An acid acceptor is generally present during the reaction for example a tertiary amine which may be a further mole of the N-benzyl derivative of the compound of the formula (XVII).

After completion, the reaction mixture may be diluted with ether, filtered and the filtrate evaporated.

The compound of formula (XVII) may be prepared by reacting the hereinbefore defined compound of formula (X), or an N-benzyl or N-$N^1$-dibenzyl derivative thereof, with the hereinbefore defined compound of formula (XVIII).

In a preferred form of the process to prepare a compound of formula (XVI), the compound of formula (XVII) is not isolated, the compound of formula (XVII) being reacted <u>in-situ</u> with a compound of formula (XVIII); and in a particularly preferred form of the process for compounds wherein Q = Q′ the compound of formula (X) is reacted with at least two molar equivalents of a compound of formula (XVIII).

In a further aspect of the process of the invention, the compounds of formula (I) may also be prepared from the hereinbefore defined compounds of formula (XI) by either;

(A) for compounds of formula (I) wherein $T^1$ represents hydrogen, reacting the compound of formula (XI) with a compound of formula (XIX):

$$Q'-X_1-\underset{\substack{|\\ \mathrm{OH}}}{\mathrm{CH}}-CH_2-R^{10} \qquad \text{(XIX)}$$

wherein Q′, $X^1$ and $R^{10}$ are as defined in relation to formula (XVIII)

or; (B) for compounds of formula (I) wherein $T^1$ represents hydrogen or a group of the above defined formula (b), carrying out the required number of the

above defined process steps (i), (ii), (iii), (iv), (v), (vi) or (vii); and if necessary therafter forming a pharmaceutically acceptable salt of the compound of formula (I).

Suitably, $R^{10}$ is a bromine atom or a tosylate group, preferably a tosylate group.

The preceding reaction between compounds (XI) and (XIX) and between compounds (VI) and (VIA) (in the above defined process step (iii), wherein $T^8$ in compound (VIA) represents a moiety of formula (j) wherein $R^x$ represents $R^{10}$) may conveniently be carried out in dimethylsulphoxide at a temperature of 50°C.

In a preferred form of the process the compound of formula (XI) is prepared by reaction of the hereinbefore defined compound of formula (X) with the hereinbefore defined compound of formula (XIX); the compound of formula (XI) is then reacted in-situ with a further compound of formula (XIX) to provide those compounds of formula (I) wherein $T^1$ is hydrogen.

In a further preferred form of the process the compounds of formula (I), wherein $T^1$ is hydrogen and $R^A = R^C$ are prepared by reaction between the hereinbefore defined compound of formula (X) and two molar equivalents of the hereinbefore defined compound of formula (XIX). Similarly, those compounds of formula (I) wherein one or both variables $T^1$ represent groups of the, above defined, formula (b) may be prepared from the compound of formula (X) and 3 or 4 molar equivalents of compound (XIX) respectively.

The compounds of formula (I) wherein $R^6$ is hydrogen may be prepared from a compound of formula (XX):

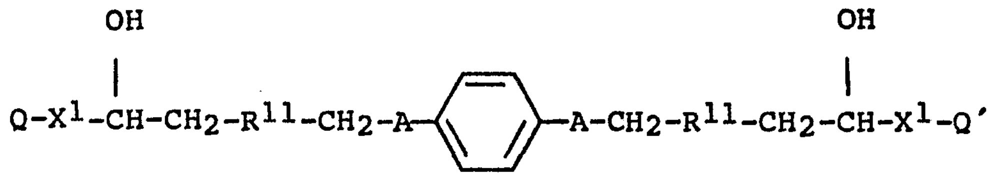

(XX)

wherein Q, $X^1$ and A are as defined in relation to formula (I) and Q′ is as defined in relation to formula (III); $R^{11}$ is a group of formula $-N{=}CR^5-$ or $-NH-C(OH)R^5-$ wherein $R^5$ is as defined in relation to formula (I) by either:

(A) for compounds of formula (I) wherein $T^1$ represents a hydrogen atom, reducing the compound of formula (XX); or (B) for compounds of formula (I) wherein $T^1$ represents hydrogen or a group of the, hereinbefore defined, formula (b), reducing the compound of formula (XX) and thereafter carrying out the required number of process steps (i), (ii), (iii), (iv), (v), (vi) and (vii);

and if necessary thereafter forming a pharmaceutically acceptable salt of the compound of formula (I).

The reduction of the compound of formula (XX) to a compound of formula (I) proceeds via an intermediate compound of formula (XXA):

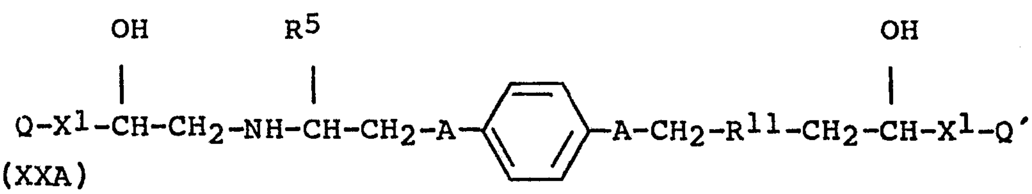

(XXA)

wherein Q, Q′, $X^1$, A, $R^5$ and $R^{11}$ are as defined in relation to formula (XX).

The compounds of formula (XXA) are generally not isolated, they are normally reduced in-situ to a compound of formula (I).

The reduction of the compounds of formula (XX) and (XXA) may be normally effected by catalytic hydrogenation under conditions described for the hydrogenation of a compound of formula (XIII).

The reduction of the compounds of the formula (XX) and (XXA) may also be effected using a complex hydride, suitably sodium borohydride, under conditions described for the reduction of a compound of formula (XIII).

The compound of formula (XX) may be prepared by reacting a compound of formula (XXI):

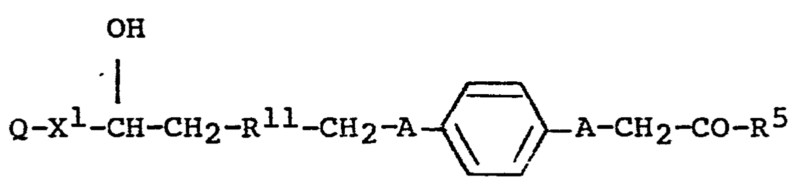

(XXI)

wherein Q, $X^1$, A and $R^{11}$ are as defined in relation to formula (XX) and $R^5$ is H or $CH_3$, with a compound of formula (XXII):

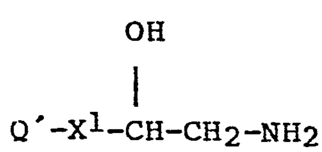

(XXII)

wherein Q′ and $X^1$ are as defined in relation to formula (XX).

Suitably, the preceding reaction is carried out under the same conditions as those described in the reaction between the hereinbefore defined compounds of formula (XIV) and (XV).

The compound of formula (XXI) may be prepared by reacting the hereinbefore defined compound of formula (XXII) with a compound of formula (XXIII):

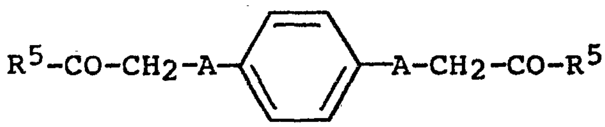
(XXIII)

wherein $R^5$ and A are as defined in relation to formula (XXI).

In a preferred form of the process to prepare a compound of formula (XX), the compound of formula (XXI) is not isolated; the compound of formula (XXI) being reacted in-situ with a compound of formula (XXII); in a particularly preferred form of the process for compounds wherein Q = Q′ the compound of formula (XXIII) is reacted with at least two molar equivalents of a compound of formula (XXII).

The compounds of formula (I) may also be prepared from a compound of formula (XXIV):

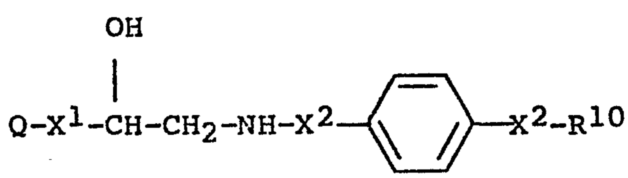
(XXIV)

wherein Q, $X^1$ and $X^2$ are as defined in relation to formula (I) and $R^{10}$ is as defined in relation to formula (XVIII) by either;
(A) for compounds of formula (I) wherein T represents a hydrogen atom, by reaction of compound (XXIV) with a compound of the, hereinbefore defined, formula (XXII);
(B) for compounds of formula (I) wherein $T^1$ represents hydrogen or a group of the, hereinbefore defined, formula (b), by reaction of compound (XXIV) with compound (XXII) and thereafter carrying out the required number of process steps (i), (ii), (iii),

(iv), (v), (vi) or (vii); and if necessary thereafter forming a pharmaceutically acceptable salt of the compound of formula (I).

Suitably, the reaction between compounds (XXII) and (XXIV) is carried out under the same conditions as those described in the reaction between the hereinbefore defined compounds of formula (XI) and (XIX).

The compounds of formula (XXIV) may be prepared by reacting a compound of formula (XXV):

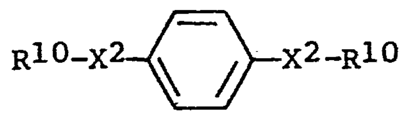

(XXV)

wherein $R^{10}$ and $X^2$ are as defined in relation to formula (XXIII) with the hereinbefore defined compound of formula (XXII).

In a preferred form of the process to prepare a compound of formula (I) wherein $T^1$ represents hydrogen, the compound of formula (XXIV) is not isolated; the compound of formula (XXIV) being reacted in-situ with a compound of formula (XXII); and in a particularly preferred form of the process the compound of formula (XXV) is reacted with two molar equivalents of a compound of formula (XXII).

In a preferred form of the process of the invention, the compounds of formula (I), wherein only one variable $T^1$ represents the above defined moiety (b), or pharmaceutically acceptable salts thereof, may be prepared by reducing a compound of formula (XXVI)

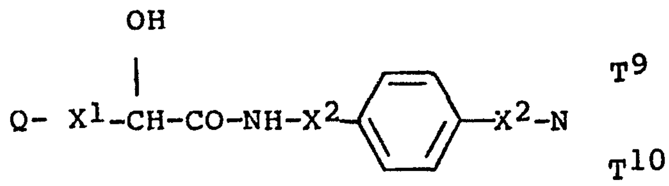

(XXVI)

wherein Q and $X^1$ are as defined in relation to formula (I), $X^2$ is as defined in relation to formula (II), and $T^9$ and $T^{10}$ each independently represent a moiety (b); and thereafter separating the required product and if required forming a pharmaceutically acceptable salt thereof.

A compound of formula (XXVI) may be prepared by reacting a compound of the hereinbefore defined formula (VIIB) with a compound of the hereinbefore defined formula (XII); suitably for compounds of formula (XXVI) wherein $T^9 = T^{10}$ the compound of formula (VIII) may be treated with two molar equivalents of the compound of formula (XII) and thereafter separating the required product.

The reduction of the compound of formula (XXVI) may be carried out using the conditions described herein for the reduction of the compound of formula (VII).

The reaction between the compounds of formula (VIIB) and (XII) may be carried out using the conditions described herein for the reaction between the compounds of formula (XI) and (XII).

It will be appreciated that the compounds of formula (VI) encompass the compounds of formula (VII), (VIIB), (VIII), (X), (XI), (XIII), (XIV), (XVI), (XVIA), (XVII), (XXIV) and (XXVI).

It will also be appreciated that the compounds of formula (VIA) encompass the compounds of formula (IX), (XII), (XV), (XVIII) and (XIX).

The hereinbefore defined compounds of formulas: (IX), (X), (XA), (XII), (XV), (XVIII), (XIX), (XXII), (XXIII) and (XXV) are known compounds and may be prepared by methods conventional in the art.

The compounds of formula (A) may also be prepared by a process analogous to any of those herein described for the preparation of the compounds of formula (I). Intermediate compounds used in the above processes for the preparation of the compounds of formula (A) may be prepared by analogous processes to any of those herein described for the preparation of the corresponding intermediate compounds to the compounds of formula (I).

In a further aspect the present invention provides a compound of formula (I) for use as an active thereaputic substance.

In a preferred aspect the present invention provides a compound of formula (I) for use in the treatment of obese and/or hyperglycaemic humans or non-human animals.

The present invention also provides a pharmaceutical composition which comprises an effective non-toxic amount of a compound of formula (I) or (VIIB), or a pharmaceutically acceptable salt thereof, and a pharmaceutically acceptable carrier therefor .

The compositions of this invention will normally be formulated for oral administration although compositions formulated for non-oral modes of administration, for example, injection, are also envisaged.

Particularly suitable oral dosage forms are unit dose forms such as tablets or capsules. Other fixed unit dose forms such as powders presented in sachets may also be used.

In accordance with conventional pharmaceutical practice the carrier may comprise a diluent, binder, filler, disintegrant, wetting agent, lubricant, colourant, flavourant or the like.

Typical carriers may therefore comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 1000 mg, more usually 1 to 800 mg and favourably 3 to 500 mg. Such doses may be taken one to six times a day in a manner such that the total daily dose for a 70 kg adult will generally be about 0.5 to 2000 mg and more usually about 5 to 1000 mg.

In addition to use in human medicine the compositions of this invention may be used to treat obesity in domestic mammals such as dogs. In general in such treatment administration to domestic mammals may be by mouth and will usually take place one or two times a

day at about 0.01 mg/kg to 25 mg/kg, for example 0.1 mg/kg to 5 mg/kg.

In a further aspect the present invention also provides a method for improving the weight gain and/or improving the feed utilisation efficiency and/or increasing lean body mass and/or decreasing birth mortality rate and increasing post-natal survival rate; of livestock, which method comprises the administration to livestock of a compound of formula (A) or (VIIB) or a veterinarily acceptable salt thereof.

Whilst the compounds of formula (A) or (VIIB) and the veterinarily acceptable salts thereof may be administered to any livestock in the abovementioned method, they are particularly suitable for improving the weight gain and/or feed utilisation efficiency and/or lean body mass in poultry, especially turkeys and chickens, cattle, pigs and sheep.

In the abovementioned method the compounds of formula (A) or (VIIB) and the veterinarily acceptable salts thereof will normally be administered orally although non-oral modes of administration, for example injection or implantation, are also envisaged. Suitably the compounds are administered in the feed-stuff or drinking water provided for the livestock. Conveniently these are administered in the feed-stuff at from $10^{-3}$ppm - 500ppm of total daily feed intake, more usually 0.01ppm to 250 ppm, suitably less than 100ppm.

The particular formulations used will of course depend upon the mode of administration but will be those used conventionally in the mode of administration chosen.

For administration in feed-stuff the drugs are conveniently formulated as a premix in association with a suitable carrier.

Accordingly, the present invention also provides a veterinarily acceptable premix formulation comprising a compound of formula (A) or (VIIB) or a veterinarily acceptable salt thereof in association with a veterinarily acceptable carrier.

Suitable carriers are inert conventional agents such as powdered starch. Other conventional feed-stuff premix carriers may also be employed.

No toxicological effects are indicated when a compound of formula (A) or of formula (I) or (VIIB) is administered in any of the dosage ranges mentioned herein.

The following Examples illustrate the invention but do not limit it in any way.

EXAMPLE 1

(R,R,R,R)-α,α'-[1,4-Phenylenebis[(1-methyl-2,1-ethanediyl) imino]methylene]bis[benzenemethanol]

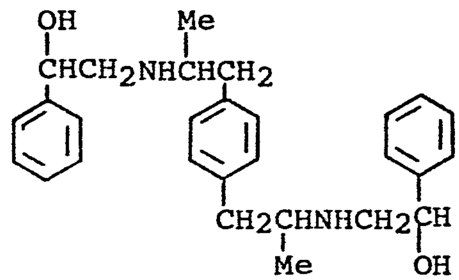

Dicyclohexylcarbodiimide (1.88g) in dry dimethyl formamide was added to a stirred solution of 1-hydroxybenzotriazole (1.23g), (R)-mandelic acid (1.39g) and (R,R)-α,α'-dimethyl-1,4-benzenediethanamine (0.88g) in dry dimethylformamide at 0°C. The mixture was allowed to stand at ambient temperature for 16h. The solvent was evaporated, the residue taken up in ethyl acetate and the solution filtered. The filtrate was washed successively with sodium carbonate solution, 2N hydrochloric acid, sodium carbonate, water (twice), dried ($MgSO_4$), filtered and evaporated to give a foam which was chromatographed on silica. Elution with methanol/chloroform (2:98) gave the bis-amide

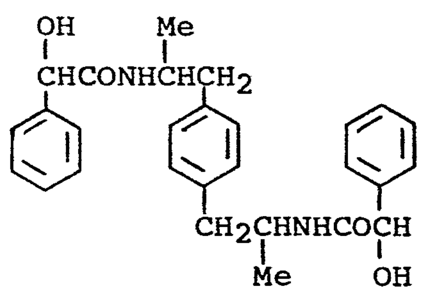

1.64g as a white foam.

$^1H$ nmr δ($CDCl_3$)

1.0 (6H,d), 2.4-2.7 (4H,m), 4.0 (2H,m), 4.6 (2H,s), 4.9 (2H,s), 6.58 (2H,d), 6.8 (4H,s), 7.1 (10H,s).

The above bis-amide (1.64g) in dry tetrahydrofuran was added dropwise under nitrogen to a stirred suspension of lithium aluminium hydride (0.3g) in ether. The suspension was refluxed for 1h at the end of the addition. The reaction mixture was cooled in ice and water (0.3ml) and more water (0.9ml) was added. The reaction mixture was filtered and evaporated. The crude reaction product contained a significant amount of starting material together with the corresponding mono-reduced material. This crude material was treated again with lithium aluminium hydride (0.4g) in the manner described above to give a yellow oil, the t.l.c. of which showed two major components. The oil was chromatographed on silica. Elution with methanol/chloroform (2:98) gave the mono amide

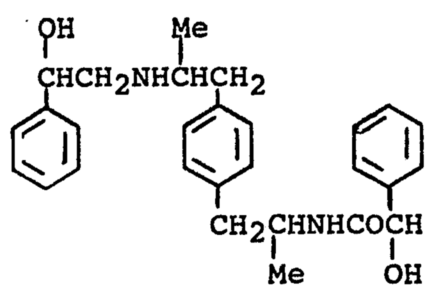

as a white solid, 0.68g, m.p. 133-135° (ethyl acetate), $[\alpha]D^{25}$(MeOH), -41.6°. Further elution with the same solvents (6:94 to 9:91) gave (R,R,R,R)-α,α'-[1,4-phenylenebis [(1-methyl-2,1-ethanediyl)imino]-methylene]bis [benzememethanol] as a white solid 0.64g, m.p. 119-122° (ethyl acetate), $[\alpha]D^{25}$ (MeOH), -53.9°.

$^1H$ nmr $\delta$($d_6$-DMSO)
0.89 (6H,d), 1.5-1.9 (2H, broad, disappears with $D_2O$), 2.38 (2H,dd), 2.65 (6H,m), 2.8 (2H,m), 4.56 (2H,t), 5.17 (2H, broad, disappears with $D_2O$), 7.01 (4H,s), 7.2-7.35 (10H,m).

EXAMPLE 2

(R,R,R,R)-α,α'-[1,4-Phenylene bis[(1-methyl-2,1-ethanediyl)imino]methylene]bis[3-chlorobenzenemethanol]

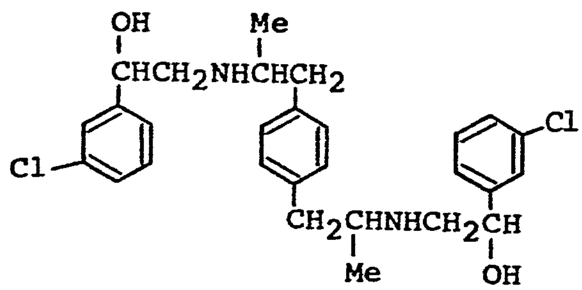

Dicyclohexylcarbodiimide (2.06g) in dry dimethylformamide was added to a stirred solution of 1-hydroxybenzotriazole (1.35g), (R)-3-chloromandelic acid (1.86g), and (R,R)-α,α'-dimethyl-1,4-benzenediethanamine (0.96g) in dry dimethylformamide at $0^{o}C$. The mixture was allowed to stand at ambient temperature for 16h. The solvent was evaporated, the residue taken up in ethyl acetate and the solution filtered. The filtrate was washed successively with 2N hydrochloric acid, sodium carbonate solution and water (twice), dried ($MgSO_4$), filtered and evaporated to give the bis amide as a white foam (2.67g) which was not purified further.

Borane dimethyl sulphide (1.9ml) was added under nitrogen to a stirred solution of this material (2.67g) in dry tetrahydrofuran and the mixture was heated under reflux for 5h. The solution was cooled, methanol (5ml) added and the solution left to stir at ambient temperature for 16h. Dry hydrogen chloride was bubbled

through the solution at 0°C and the mixture then heated under reflux for 1h during which time a solid precipitated. The solvent was evaporated, the residue partitioned between 2N sodium hydroxide solution and ethyl acetate, the organic layer separated, dried ($MgSO_4$) and evaporated to give an oil which was chromatographed on alumina. Elution with methanol-chloroform (2:98-6:94) gave (R,R,R,R)-α,α'-[1,4-phenylene bis[(1-methyl-2,1-ethanediyl)imino] methylene] bis[3-chlorobenzenemethanol) as a white solid, 1.0g, m.p. 120-123° (ethyl acetate), $[\alpha]_D^{25}$(MeOH), -54.0°.

$^1H$ nmr δ($d_6$-DMSO+$D_2O$)

0.21 (6H,d), 2.41 (2H,dd), 2.64-2.75 (6H,m), 2.85 (2H,m), 4.61 (2H,t), 7.02 (4H,s), 7.2-7.35 (6H,m), 7.39 (2H,s).

EXAMPLE 3

(R,R)-α-α'-[1,4-Phenylenedioxybis [(2,1-ethanediyl)-imino] methylene] bis [benzenemethanol]

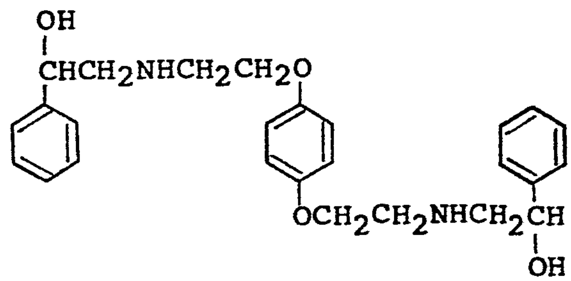

This compound was prepared by a similar method to that described in Example 2 using (R)-mandelic acid and 1,4-benzenedioxyethanamine except that after treatment with borane methyl sulphide the reaction mixture was heated under reflux for 16h before being quenched with methanol. The solution was then heated under reflux for 1h, cooled, treated with hydrogen chloride and worked up as described in Example 2. Chromatography on silica and elution with methanol-chloroform (6:94) gave (R,R)-α,α'-[1,4-phenylenedioxybis [(2,1-ethanediyl) imino] methylene] bis [benzenemethanol] as a white solid, 1.0 g, m.p. 144-147° (ethyl acetate), $[\alpha]D^{25}$ (MeOH), -37.5°.

$^1H$ nmr δ($d_6$-DMSO)

1.8-2.1 (2H, broad, disappears with $D_2O$), 2.70 (4H,d), 2.90 (4H,t), 3.95 (4H,t), 4.64 (2H,t), 5.28 (2H, broad, disappears with $D_2O$), 6.84 (4H,s), 7.2-7.5 (10H,m).

EXAMPLE 4

(R,R)-α,α'-[1,4-Phenylenebis [(2,1-ethanediyl)imino]-methylene]bis[benzenemethanol]

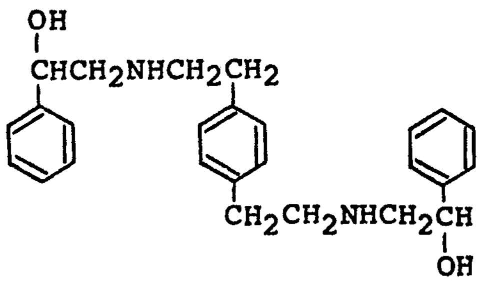

This compound was prepared by a similar method to that described in Example 3 using (R)-mandelic acid and 1,4-benzenediethanamine. (R,R)-α,α'-[1,4-phenylenebis [(2,1-ethanediyl)imino]methylene]bis[benzenemethanol] was obtained as a white solid, m.p. 143-145$^o$ (ethyl acetate), $[\alpha]D^{25}$ (MeOH) -38.4$^o$.

$^1H$ nmr δ($d_6$-DMSO)

1.6 (2H, broad, disappears with $D_2O$), 2.6-2.72 (8H,m), 2.72-2.82 (4H,m), 4.60 (2H,t), 5.20 (2H, broad, disappears with $D_2O$), 7.09 (4H,s), 7.15-7.35 (10H,m).

EXAMPLE 5

(R,R)-α,α[1]-[1,4-Phenylenedioxybis[(2,1-ethanediyl)-imino]methylene]bis[3-chlorobenzene methanol]

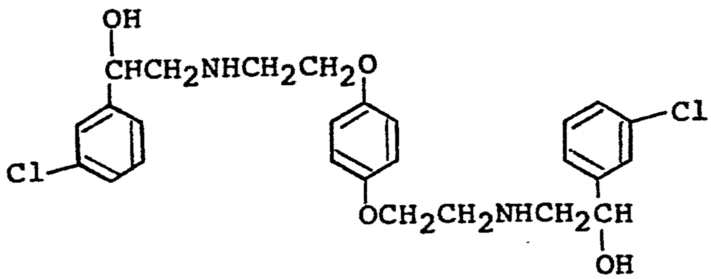

This compound was prepared by the method described in Example 3 using (R)-3-chloromandelic acid and 1,4-benzenedioxyethanamine.

(R,R)-α,α[1]-[1,4-phenylenedioxybis[(2,1-ethanediyl)-imino]methylene]bis[3-chlorobenzene methanol] was obtained as a white solid, m.p. 119-122° (ethyl acetate), $[\alpha]D^{25}$ (MeOH) -39.7°.

$^1H$ nmr $\delta(d_6\text{-DMSO})$

2.6 (4H,d), 2.8 (4H,t), 2.8-3.5 (2H, broad, disappears with $D_2O$), 3.9 (4H,t), 4.6 (2H,t), 5.4 (2H, broad, disappears with $D_2O$), 6.88 (4H,s), 7.35 (6H,m), 7.4 (2H,s).

EXAMPLE 6

(R,R)-α,α'-[1,4-Phenylenebis[(2,1-ethanediyl]imino]-methylene]bis[3-chlorobenzene methanol]

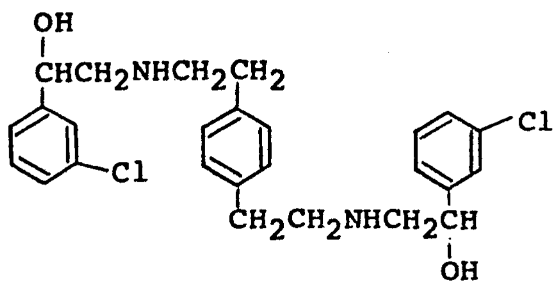

This compound was prepared by a similar method to that described in Example 3 using (R)-3-chloromandelic acid and 1,4-benzenediethanamine.

(R,R)-α,α'-[1,4-phenylenebis [(2,1-ethanediyl)imino]-methylene]bis[3-chlorobenzene methanol] was obtained as a white solid, m.p. 160-163$^o$ (ethyl acetate). A second crop (m.p. 115-118$^o$) having an identical $^1H$ nmr spectrum was also obtained.

$^1H$ nmr δ($d_6$-DMSO)

1.6-1.9 (2H, broad, disappears with $D_2O$), 2.59-2.68 (8H,m), 2.72-2.78 (4H,m), 4.62 (2H,t), 5.4 (2H, broad, disappears with $D_2O$), 7.09 (4H,s), 7.26-7.34 (6H,m), 7.38 (2H,s).

EXAMPLE 7

α,α'-[1,4-Phenylenedioxybis [(1-methyl-2,1-ethanediyl) imino]methylene]bis[(R)-3-chlorobenzenemethanol]

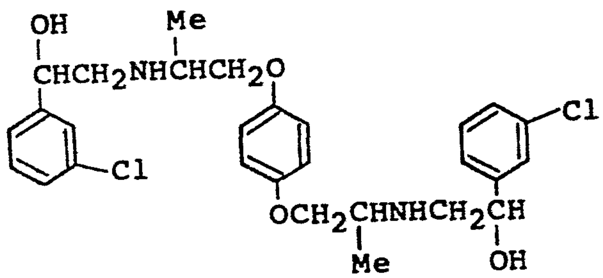

This compound was prepared by a similar method to that described in Example 2 using (R)-3-chloromandelic acid and α,α'-dimethyl-1,4-benzene dioxyethanamine (20% ee of the (R)-enantiomer).

α,α'-[1,4-phenylenedioxybis[(1-methyl-2,1-ethanediyl)-imino] methylene] bis [(R)-3-chlorobenzenemethanol] was obtained as a mixture of diastereoisomers as a white solid, m.p. 131-136° (ethyl acetate), $[\alpha]D^{25}$ (MeOH), -29.6° (c=0.45).

$^1H$ nmr δ($d_6$-DMSO)

1.1 (6H,d), 2.72 (4H,d), 2.95 (2H,q), 3.28 (2H, broad, disappears with $D_2O$), 3.72 (4H,d), 4.64 (2H,t), 5.5 (2H, broad, disappears with $D_2O$), 6.88 (4H,s), 7.34 (6H,s), 7.4 (2H,s).

$^{13}C$ nmr ppm ($d_6$-DMSO)

(17.02, 17.06), (51.31, 51.54), (54.02, 54.35), (70.93, 71.12), (72.56, 72.60), 115.53, (124.53, 124.61), (125.67, 125.70), 126.71, 129.83, 132.76, (146.85, 146.90), (152.64, 152.70).

Numbers in parentheses indicate paired signals in the ratio of 60:40.

EXAMPLE 8

(R,R)-α,α'-[1,2-Phenylenebis[(2,1-ethanediyl)imino] methylene] bis [3-chlorobenzenemethanol], dihydrochloride

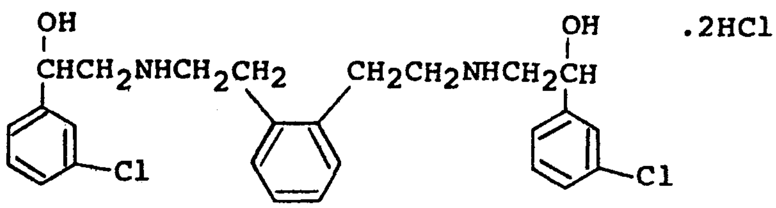

This compound was prepared by a similar method to that described in Example 3 using (R)-3-chloromandelic acid and 1,2-benzenediethanamine. After chromatography an ethereal solution of the resulting oil was treated with hydrogen chloride to give (R,R)-α,α'-[1,2-phenylenebis [(2,1-ethanediyl)imino] methylene] bis [3-chlorobenzenemethanol], dihydrochloride as a white solid, m.p. 227-231° (ethyl acetate - ethanol), $[\alpha]D^{25}$ (MeOH) -25.4° (c=0.25).

$^{1}H$ nmr δ($d_6$-DMSO)

3.0-3.4 (10H,m), 3.26 (2H,dd), 5.07 (2H,dd), 6.32 (2H, bs, disappears with $D_2O$), 7.24 (4H,s), 7.35-7.44 (6H,m), 7.46 (2H,s), 9.0-9.5 (4H, broad, disappears with $D_2O$).

EXAMPLE 9

(R,R,R,R)-α,α'-[1,4-Phenylenebis [(1-methyl-2,1-ethanediyl)imino] methylene] bis [3,4-dichlorobenzenemethanol]

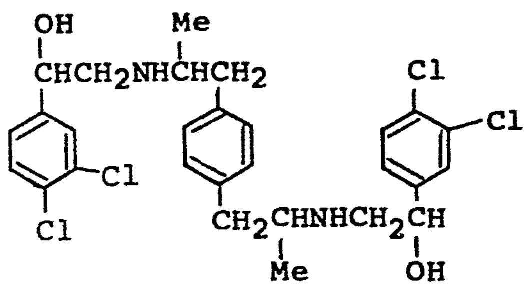

This compound was prepared by a similar method to that described in Example 3 using (R)-3,4-dichloromandelic acid and (R,R)-α,α'-dimethyl-1,4-benzenediethanamine. (R,R,R,R)-α,α'-[1,4-phenylenebis[(1-methyl-2,1-ethanediyl)imino] methylene] bis [3,4-dichlorobenzenemethanol] was obtained as a white solid, m.p. 124-126° (ethyl acetate).

$^1H$ nmr δ($d_6$-DMSO)

0.88 (6H,d), 2.38 (2H,dd), 2.6-2.8 (8H), 4.57 (2H,t), 5.38 (2H, s, disappears with $D_2O$), 6.98 (4H,s), 7.29 (2H,dd), 7.51-7.54 (4H,m).

EXAMPLE 10

(R,R,R,R)-1,1'-[1,4-Phenylenebis [(1-methyl-2,1-ethanediyl)imino]] bis [3-phenoxypropan-2-ol], dihydrochloride

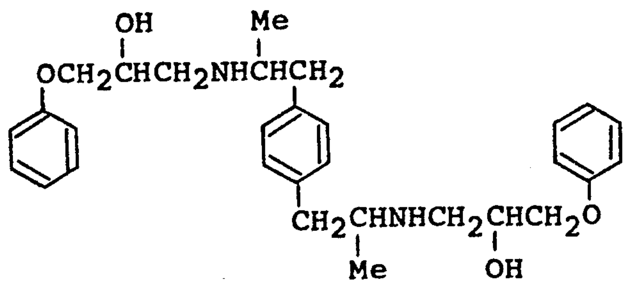

.2HCl

A mixture of (R)-phenoxymethyloxirane (0.39g) and (R,R)-α,α'-dimethyl-1,4-benzenediethanamine (0.25g) in ethanol (50ml) was stirred and heated under reflux for 3 days. Evaporation of the solvent gave a colourless oil which was chromatographed on silica. Elution with chloroform-methanol (96:4) gave a small amount of (R,R,R,R,R)-1-[2-[4-[2-[bis (2-hydroxy-3-phenoxypropyl) amino] propyl]phenyl]-1-methylethylamino]-3-phenoxypropan-2-ol. Change of solvent to chloroform-methanol (90:10) gave an oil which was treated with hydrogen chloride to give (R,R,R,R)-1,1'-[1,4-phenylenebis [(1-methyl-2,1-ethanediyl)imino]] bis [3-phenoxypropan-2-ol], dihydrochloride, (0.1g), m.p. 187-189° (EtOAc/MeOH).

$^1$H nmr δ($d_6$-DMSO)

1.12 (6H,d), 2.63 (2H,dd), 3.15 (2H,dd), 3.22-3.3 (4H,m), 3.3-3.55 (2H,m), 3.99 (4H,d), 4.24 (2H,m), 5.88 (2H,s, disappears with $D_2O$), 6.93-6.98 (6H,m), 7.22 (4H,s), 7.22-7.34 (4H,m), 8.5-9.5 (4H, broad, disappears with $D_2O$).

Change of solvent to acetonitrile-methanol-ammonia (15:1:0.5) gave a small amount of (R,R,R)-1-[2-[4-(2-aminopropyl)phenyl]-1-methylethylamino]-3-phenoxypropan-2-ol.

EXAMPLE 11

1,1'-[1,4-Phenylenebis [((R)-1-methyl-2,1-ethanediyl) imino]]bis [3-phenoxypropan-2-(S)-ol], dihydrochloride

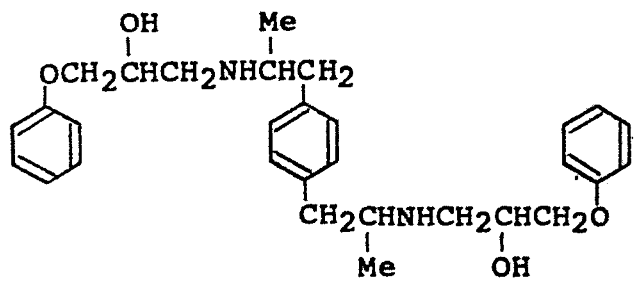

.2HCl

A mixture of (S)-phenoxymethyloxirane (0.43g) and (R,R,R,R)-N,N'-(di-α-methylphenylmethyl)-α,α'-dimethyl -1,4-benzenediethanamine (0.57g) in isopropanol (50ml) was stirred and heated under reflux for 3 days. The solvent was evaporated and the residual oil was chromatographed. Elution with chloroform gave 1,1'-[NN'-[(R)-di-α-methylphenylmethyl)-[1,4-phenylenebis[((R)-1-methyl-2,1-ethanediyl)imino]]-bis [3-phenoxypropan-2-(S)-ol]], (0.2g) followed by 1-[[[2-[4-[2-[(N-(R)-α-methylphenylmethyl)amino] propyl]phenyl]-1-methylethyl]-(N-(R)-α-methylphenylmethyl)] amino]-3-phenoxypropan-2-(S)-ol, 0.3g, followed by unreacted starting amine.

A solution of 1,1'-[NN'-[(R)-di-α-methylphenylmethyl)-[1,4-phenylenebis [((R)-1-methyl-2,1-ethanediyl)imino]] bis [3-phenoxypropan-2-(S)-ol]], dihydroochloride (0.2g) in methanol containing 10% palladium on charcoal was hydrogenated at ambient temperature and pressure. The catalyst was filtered off and the solvent evaporated to give 1,1'-[1,4-phenylenebis[((R)-1-methyl -2,1-ethanedily)imino]] bis [3-phenoxypropan-2-(S)-ol], dihydrochloride as a white solid, (0.065g), m.p. 196-200° (EtOAc/MeOH).

$^1H$ nmr δ($d_6$-DMSO)

1.09 (6H,d), 2.63 (2H,t), 3.08 (2H,t), 3.1-3.6 (6H,m), 4.00 (4H,d), 4.23 (2H,m), 5.90 (2H, broad, disappears with $D_2O$), 6.95 (6H,m), 7.22 (4H,s), 7.31 (4H,t), 8.1-9.0 (4H, broad, disappears with $D_2O$)

EXAMPLE 12

(R,R,R)-3,4-Dichloro-α-[[[2-[4-[2-[[2-(3-chlorophenyl)-2-hydroxyethyl] amino] ethyloxy] phenyl]-1-methylethyl] amino] methyl] benzenemethanol, hemihydrate

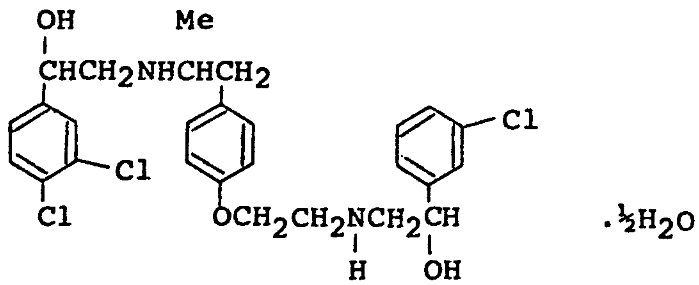

Dicyclohexylcarbodiimide (0.45g) was added to a stirred solution of (R)-3-chloromandelic acid (0.41g), 1-hydroxybenzotriazole (0.3g) and (R,R)-3,4-dichloro-α-[[[2-[4-[2-[amino] ethyloxy] phenyl]-1-methylethyl] amino] methyl] benzenemethanol (0.84g) in dry dimethylformamide (50ml) and the reaction mixture left for 18h. The product amide (0.84g) was obtained according to the procedure described in Example 1 and treated with borane-methyl sulphide complex (0.7g = 0.88ml) according to the procedure described in Example 3. (R,R,R)-3,4-Dichloro-α-[[[2-[4-[2-[[2-(3-chlorophenyl)-2-hydroxyethyl] amino] ethyloxy] phenyl]-1-methylethyl] amino] methyl] benzenemethanol, hemihydrate (0.1g) was obtained as a white solid, m.p. 70-74° (hexane-acetone).

$^1$H nmr δ($d_6$-DMSO)

0.89 (6H,d), 1.5-2.0 (2H, broad, disappears with $D_2O$), 2.37 (1H,dd), 2.61-2.72 (6H,m), 2.89 (2H,t), 3.38 (1H,t), 3.96 (2H,t), 4.30 (½$H_2O$, disappears with $D_2O$), 4.57 (1H,t), 4.65 (1H,t), 5.4 (2H, broad, disappears with $D_2O$), 6.79 (2H,d), 7.01 (2H,d), 7.25-7.34 (4H,m), 7.40 (1H,m), 7.52-7.55 (2H,m).

EXAMPLE 13

(R,R,R,R)-α,α[1]-[1,4-Phenylenebis [(1-methyl-2,1-ethanediyl)imino]methylene] bis [4-hydroxybenzene-methanol]

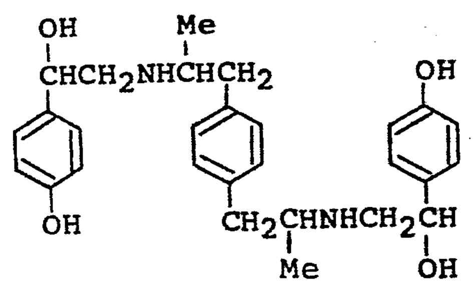

(R,R,R,R)-α,α[1]-[1,4-Phenylenebis [(1-methyl-2,1-ethanediyl)imino]methylene] bis [4-(phenylmethyloxy)benzene-methanol] (0.5g) in methanol (50ml) containing 10% palladium on carbon was hydrogenated at ambient temperature and pressure until hydrogen uptake ceased. The mixture was filtered and the filtrate evaporated to give a glass which was chromatographed on silica. Elution with acetonitrile:methanol:ammonia (40:4:1) gave (R,R,R,R)-α,α[1]-[1,4-Phenylenebis [(1-methyl-2,1-ethanediyl)imino]methylene] bis [4-hydroxybenzene-methanol] as a white solid, (0.18g), m.p. 130-135° (EtOAc).

$^1H$ nmr $\delta(d_6$-DMSO)

0.88 (6H,d), 2.37 (2H,dd), 2.59-2.69 (6H,m), 2.78 (2H,dd), 3.0-3.5 (2H, broad, disappears with $D_2O$), 4.44 (2H,t), 4.75-5.25 (2H, broad, disappears with $D_2O$), 6.68 (4H,d), 7.01 (4H,s), 7.08 (4H,d), 8.75-9.5 (2H, broad, disappears with $D_2O$).

EXAMPLE 14

α,α[1]-[1,4-Phenylenebis[((R)-1-methyl-2,1-ethanediyl) imino]methylene] bis [(4-amino-3,5-dichloro)benzene-methanol]

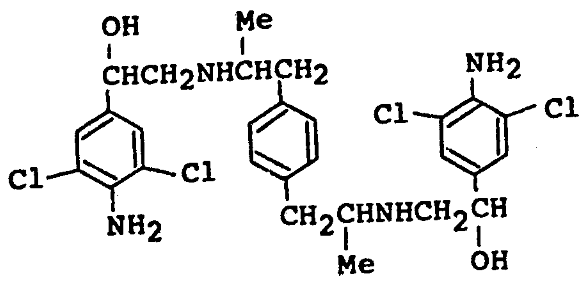

A mixture of (4-amino-3,5-dichloro)phenylglyoxal, ethyl hemiacetal (2.06g) and (R,R)-α,α[1]-dimethyl-1,4-benzenediethanamine (0.75g) was stirred and heated under reflux for 3h in benzene using a Dean and Stark head. The resulting yellow solid (1.4g) was filtered and dried under vacuum. This material was suspended in methanol containing platinum oxide and hydrogenated at ambient temperature and pressure. The catalyst was filtered off and the filtrate evaporated to give a foam which was chromatographed on alumina. Elution with methanol-chloroform (2:98) gave α,α[1]-[1,4-phenylenebis [((R)-1-methyl-2,1-ethanediyl)imino]methylene] bis [(4-amino-3,5-dichloro)benzenemethanol] as a 1:1 mixture of diastereoisomers as a white foam which solidified (m.p. 59-63°) on trituration with hexane.

$^1H$ nmr δ($d_6$-DMSO)

0.84, 0.88 (total 6H, d+d), 1.45 (2H, bs, disappears with $D_2O$), 2.35-2.78 (10H,m), 4.41 (2H,t), 5.15 (2H,bs, disappears with $D_2O$), 5.30 (4H, s, disappears with $D_2O$), 7.02 (4H, t, collapses to singlet at 130°), 7.16 (4H,s).

0196849

EXAMPLE 15

(R,R,R,R)-3-Chloro-α-[[[2-[4-[2-[[2-(4-hydroxyphenyl)-2-hydroxyethyl]amino]propyl]phenyl]-1-methylethyl]amino]methyl]benzenemethanol

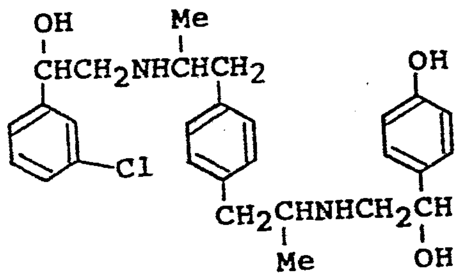

(R,R,R,R)-3-Chloro-α-[[[2-[4-[2-[[2(4-phenylmethyloxyphenyl)-2-hydroxyethyl]amino]propyl]phenyl]-1-methylethyl]amino]methyl]benzenemethanol (0.85g) was dissolved in glacial acetic acid (50ml) and hydrogenated at ambient temperature and pressure over 10% palladium on carbon catalyst until the theoretical amount of hydrogen had been absorbed. The catalyst was filtered off, the filtrate evaporated and the resulting foam chromatographed on silica. Elution with acetonitrile:methanol:ammonia (40:3:1) gave (R,R,R,R)-3-Chloro-α-[[[2-[4-[2-[[2-(4-hydroxyphenyl)-2-hydroxyethyl]amino]propyl]phenyl]-1-methylethyl]amino]methyl]benzenemethanol as a white solid containing half a molar equivalent of carbon tetrachloride, m.p. 58-60° (carbon tetrachloride).

$^1H$ nmr δ($d_6$-DMSO)

0.86 (3H,d), 0.88 (3H,d), 1.55 (2H, broad, disappears with $D_2O$), 2.3-2.45 (2H,m), 2.6-2.7 (6H,m), 2.77 (2H,m), 4.44 (1H,t), 4.57 (1H,t), 5.0 (1H, broad, disappears with $D_2O$), 5.37 (1H, broad, disappears with $D_2O$), 6.67 (2H,d), 7.00 (4H,s), 7.08 (2H,d), 7.24-7.32 (3H,m), 7.36 (1H,s), 9.25 (1H, broad, disappears with $D_2O$).

EXAMPLE 16

3-Chloro-α-(R)-[[[2-[4-[2-(R)-[(3-phenoxy-2-(S)-hydroxypropyl)amino]propyl]phenyl]-1-(R)-methylethyl]-amino]methyl]benzenemethanol, dihydrochloride

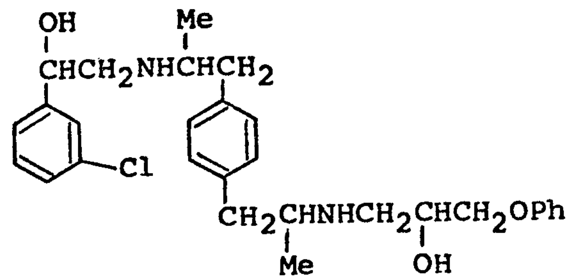

.2HCl

A solution of 3-chloro-α-(R)-hydroxy-N-[2-[4-[2-(R)-[(3-phenoxy-2-(S)-hydroxypropyl)amino]propyl]phenyl]-1-(R)-methylethyl] benzeneacetamide, Example X 9, (1.4g) in dry tetrahydrofuran (40ml) was added under nitrogen to a stirred suspension of lithium aluminium hydride (1.17g) in dry tetrahydrofuran (15ml). The reaction mixture was heated under reflux for 16h at the end of addition, cooled, water (1.2ml) followed by 2N sodium hydroxide solution added and the mixture filtered. The filtrate was evaporated to give a clear oil (1.2g) which was chromatographed on silica. Elution with methanol-chloroform (2:98 - 10:90) gave a clear oil which was treated with ethereal hydrogen chloride to give 3-chloro-α-(R)-[[[2-[4-[2-(R)-[(3-phenoxy-2-(S)-hydroxypropyl)amino]propyl]phenyl]-1-(R)-methylethyl]amino]methyl]benzenemethanol, dihydrochloride as a white solid, m.p. 207-210°, $[\alpha]D^{25}$(MeOH) -44.4°.

$^1$H nmr δ($d_6$-DMSO)

1.12 (6H,d), 2.67 (2H,t), 3.08 (2H,dq), 3.12-3.34 (6H, m), 3.46 (2H,m), 4.02 (2H,d), 4.31 (1H,m), 5.11 (1H,dd), [5.92 (1H,d) + 6.35 (1H,d), both disappear with $D_2O$], 6.97 (3H,d), 7.22 (6H,m), 7.31-7.46 (3H,m), 7.50 (1H,s), [8.8 (2H) + 9.3 (1H) + 9.6 (1H), all broad s and disappear with $D_2O$].

EXAMPLE 17

3-Chloro-α-(R)-[[[2-[4-[2-(R)-[bis(3-phenoxy-2-(S)-hydroxypropyl)amino]propyl]phenyl]-1-(R)-methylethyl]-amino]methyl]benzenemethanol, dihydrochloride

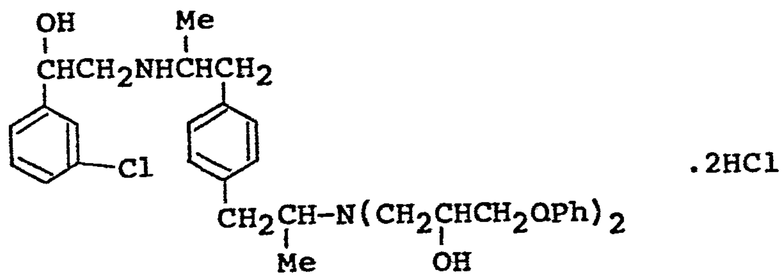

Treatment of 3-chloro-α-(R)-hydroxy-N-[2-[4-[2-(R)-[bis(3-hydroxy-2-(S)-hydroxypropyl)amino]propyl] phenyl]-1-(R)-methylethyl]benzeneacetamide (710mg) with lithium aluminium hydride (430mg) in the manner described in Example 16 gave a clear oil which was chromatographed on silica. Elution with methanol-chloroform (5:95) gave an oil which was treated with ethereal hydrogen chloride to give 3-chloro-α-(R)-[[[2[4-[2-(R)-[bis(3-phenoxy-2-(S)-hydroxypropyl)-amino]propyl]phenyl]-1-(R)-methylethyl]amino]methyl] benzenemethanol, dihydrochloride as a white solid (220mg), m.p. 85-92°.

$^1H$ nmr $\delta(d_6$-DMSO)

1.11 (3H,d), 1.21 (3H,d), 2.51 (1H,t), 2.66 (1H,t), 3.0-3.55 (11H,m), 3.8-4.2 (5H,m), 4.47 (2H, broad s), 5.10 (1H,dd), 6.08 (2H, broad, disappears with $D_2O$), 6.35 (1H, broad, disappears with $D_2O$), 6.95-7.00 (6H,m), 7.20-7.50 (12H,m), [8.88 (1H) + 9.01 (1H) + 9.52 (1H) all disappears with $D_2O$).

Further elution with the same solvent system gave a mixture of 3-chloro-α-(R)-[[[2-[4-[2-(R)-[N-(3-phenoxy-2-(S)-hydroxypropyl)-N-(2-(S)-hydroxypropyl)amino]-propyl]phenyl]-1-(R)-methylethyl]amino]methyl]benzene-methanol and 3-chloro-α-(R)-[[[2-[4-[2-(R)-[N-(3-phenoxy-2-(S)-hydroxypropyl)-N-(3-hydroxypropyl)amino]-propyl]phenyl]-1-(R)-methylethyl]amino]methyl]benzene-methanol.

EXAMPLE 18

4-Amino-3,5-dichloro-α-[[[2-[4-[2-(R)-[[2-(R)-(4-hydroxyphenyl)-2-hydroxyethyl]amino]propyl]phenyl]-1-(R)-methylethyl]amino]methyl]benzenemethanol

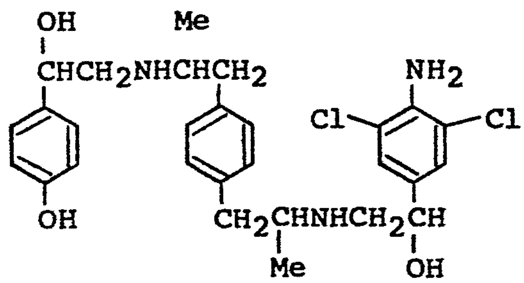

This compound was obtained as a white solid, m.p. 72-76°, from the corresponding phenylmethyloxy derivative, Example X 10, according to the procedure described in Example 15.

$^1H$ nmr δ($d_6$-DMSO)

0.88 (6H,d), 1.5 (2H, broad s, disappears with $D_2O$), 2.34-2.4 (2H,m), 2.5-2.7 (6H,m), 2.61-2.77 (2H,m), 3.3 (2H, broad s, disappears with $D_2O$), 4.42 (2H,m), 4.9 (2H, broad, disappears with $D_2O$), 6.68 (2H,d), 7.00 (2H,s), 7.03 (2H,s), 7.07 (2H,d), 7.13 (2H,s), 9.2 (1H, broad s, disappears with $D_2O$).

EXAMPLE 19

4-Hydroxy-α-(R)-[[[2-[4-[2-(R)-[(3-phenoxy-2-(S)-hydroxypropyl)amino]propyl]phenyl]-1-(R)-methylethyl]-amino]methyl]benzenemethanol

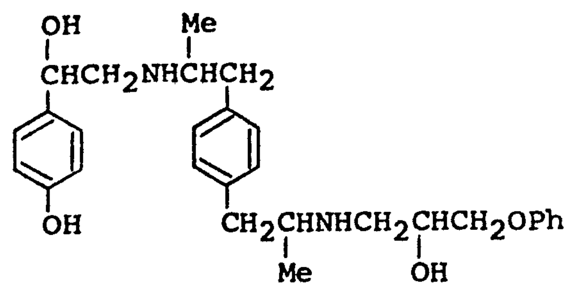

The corresponding phenylmethyloxy derivative, Example X 11, (0.4g) was hydrogenated at ambient temperature and pressure in methanol containing 10% palladium on charcoal. The catalyst was filtered off and the filtrate evaporated to give an oil which was chromatographed on silica. Elution with acetonitrile-methanol-ammonia (40:3:1) gave 4-hydroxy-α-(R)-[[[2-[4-[2-(R)-[(3-phenoxy-2-(S)-hydroxypropyl)amino]propyl]-phenyl]-1-(R)-methylethyl]amino]methyl]benzenemethanol as a white solid (0.23g), m.p. 59-63°.

$^1H$ nmr δ($d_6$-DMSO)

0.93 (6H,d), 2.08-2.4 (2H,m), 2.7-2.78 (6H,m), 2.80-2.89 (2H,m), 3.30 (2H, broad s, disappears with $D_2O$), 3.85-3.95 (3H,m), 4.53 (1H,t), 4.75-5.5 (2H, broad, disappears with $D_2O$), 6.70 (2H,d), 6.89-6.94 (3H,m), 7.03-7.13 (6H,m), 7.27 (2H,t), 9.25 (1H, broad, disappears with $D_2O$).

EXAMPLE 20

4-Hydroxy-α-(R)-[[[2-[4-[2-(R)-[bis(3-phenoxy-2-(S)-hydroxypropyl)amino]propyl]phenyl]-1-(R)-methylethyl]-amino]methyl]benzenemethanol

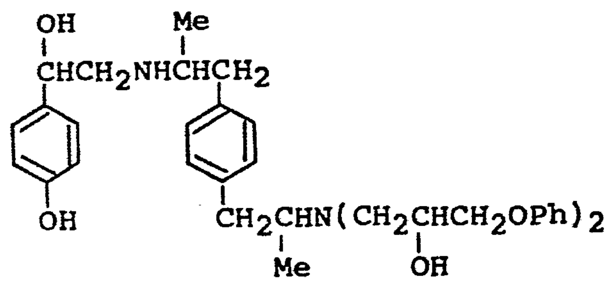

This compound was prepared from the corresponding 4-phenylmethyloxy derivative, Example X 15, following the procedure described in Example 19. Chromatography on silica and elution with methanol-chloroform gave 4-hydroxy-α-(R)-[[[2-[4-[2-(R)-[bis(3-phenoxy-2-(S-hydroxypropyl)amino]propyl]phenyl]-1-(R)-methylethyl]-amino]methyl]benzenemethanol as a white solid, m.p. 61-65°.

$^{1}H$ nmr δ($d_6$-DMSO)

0.84 (3H,d), 0.96 (3H,d), 2.32-2.47 (2H,m), 2.58-2.72 (4H,m), 2.79-2.92 (5H,m), 3.0-3.03 (1H,m), 2.25-3.0 (1H, very broad), 3.83-3.85 (4H,m), 3.92 (2H,t), 4.64 (1H,t), 4.81 (2H, broad s, disappears wtih $D_2O$), 5.25-6.0 (1H broad, disappears with $D_2O$), 6.73 (2H,d), 6.86-6.92 (6H,m), 7.07 (4H,q), 7.12 (2H,d), 7.24 (4H,t), 9.3 (1H, broad s, disappears with $D_2O$).

EXAMPLE 21

4-Amino-3,5-dichloro-α-[[[2-[4-[2-(R)-[[2-(R)-(3-chlorophenyl)-2-hydroxyethyl]amino]propyl]phenyl]-1-(R)-methylethyl]amino]methyl]benzenemethanol

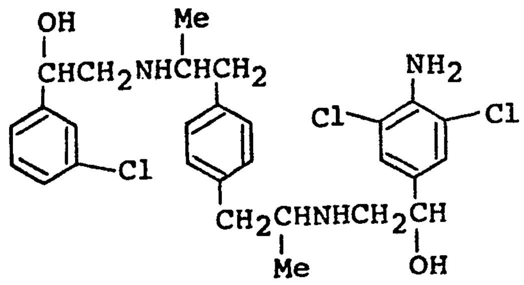

Treatment of 3-chloro-α-(R)-hydroxy-N-[2-[4-[2-(R)-[[2-(4-amino-3,5-dichlorophenyl)-2-hydroxyethyl]amino]-propyl]phenyl]-1-(R)-methylethyl]benzeneacetamide, Example X 13 with borane methyl sulphide in tetrahydrofuran for 3h at reflux and then work up following the procedure described in Example 2 gave 4-amino-3,5-dichloro-α-[[[2-[4-[2-(R)-[[2-(R)-(3-chlorophenyl)-2-hydroxyethyl]amino]propyl]phenyl]-1-(R)-methylethyl]amino]methyl]benzenemethanol as a white foam.

$^1H$ nmr δ($d_6$-DMSO)

0.88 (6H,d), 1.56 (2H, broad s, disappears with $D_2O$), 2.36-2.48 (2H,m), 2.62-2.76 (6H,m), 2.77-2.80 (2H,m), 4.40 (1H, dd), 4.55 (1H,t), [5.15 (1H), 5.30 (3H), all disappear with $D_2O$], 7.01 (2H,s), 7.02 (2H,s), 7.15 (2H,s), 7.24-7.31 (3H,m), 7.36 (1H,s).

EXAMPLE X 1

(R,R,R,R)-N,N'-(Di-α-methylphenylmethyl)-α,α[1]-dimethyl-1,4-benzenediethanamine, dihydrochloride

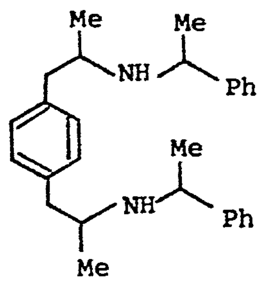

.2HCl

A mixture of (R)-α-methylphenylmethanamine (91.8g) and 1,4-benzene di-propan-2-one (72.2g) in benzene was stirred and heated under reflux for 16h using a Dean and Stark head. The solvent was evaporated, the residual oil dissolved in ethanol containing platinum oxide and the mixture hydrogenated at ambient temperature and pressure until hydrogen uptake ceased. The catalyst was filtered off and the filtrate evaporated. The residual oil was taken up in ethyl acetate and treated with hydrogen chloride gas. Two recrystallizations of the resulting solid from ethylacetate-methanol gave (R,R,R,R)-N,N'-(Di-α-methylphenylmethyl)-α,α[1]-dimethyl-1,4-benzene-diethanamine, dihydrochloride as a white solid (30.5g), m.p. 274°, $[\alpha]_D^{25}$(MeOH) + 55.6°.

$^1$H nmr δ($d_6$-DMSO)

1.07 (6H,d), 1.54 (6H,d), 2.6 (2H,m), 3.37 (4H,m), 4.59 (2H,m), 6.90 (4H,s), 7.39-7.53 (6H,m), 7.72-7.75 (4H,m), 9.44 (2H, broad, disappears with $D_2O$), 10.25 (2H, broad, disappears with $D_2O$).

EXAMPLE X 2

(R,R)-α,α¹-Dimethyl-1,4-benzenediethanamine, dihydrochloride

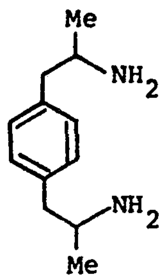

.2HCl

10% Palladium on carbon in ethanol was added to a solution of (R,R,R,R)-N,N¹-(di-α-methylphenylmethyl)-α,α¹-dimethyl-1,4-benzenediethanamine, dihydrochloride (21.0g) in methanol and the mixture hydrogenated at 50-100 psi and 50-100° until uptake of hydrogen ceased. The catalyst was filtered off and the filtrate evaporated to give (R,R)-α,α¹-Dimethyl-1,4-benzenediethanamine, dihydrochloride as a white solid, (8.58g), m.p. > 300°, $[\alpha]D^{25}$ (MeOH), -11.5°.
$^1H$ nmr δ($d_6$-DMSO)
1.15 (6H,d), 2.6-3.2 (4H,m), 3.31 (2H,m), 7.22 (4H,s), 8.3 (6H, broad, disappears with $D_2O$).

EXAMPLE X 3

(R,R,R,R)-α,α[1][1,4-Phenylenebis [(1-methyl-2,1-ethanediyl)imino]methylene] bis [4-(phenylmethyloxy)-benzene methanol]

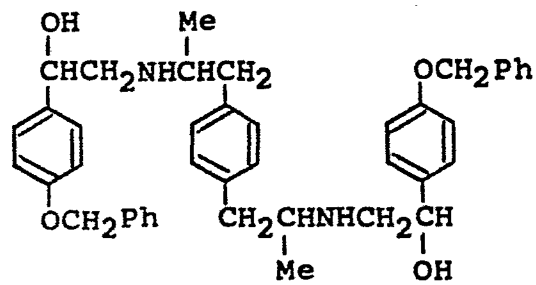

This compound was obtained by a similar procedure to that described in Example 1 using (R)-4-(phenylmethyloxy) mandelic acid and (R,R)-α,α[1]-dimethyl-1,4-benzenediethanamine. (R,R,R,R)-α,α[1][1,4-Phenylenebis [(1-methyl-2,1- ethanediyl)imino]methylene] bis [4-(phenylmethyloxy)benzene methanol] was obtained as a white solid, m.p. 128-130°.

$^1H$ nmr δ($CDCl_3$)

0.88 (6H,d), 2.3-3.0 (14H, (includes 4H which exchange with $D_2O$), m), 4.31 (2H,dd), 4.85 (4H,s), 6.8 (4H,d), 7.02 (4H,s), 7.3 (4H,d), 7.3 (10H,s).

EXAMPLE X 4

(R,R)-3,4-Dichloro-α-[[[2-[4-[2-[amino]ethyloxy]phenyl]-1-methylethyl]amino]methyl]benzenemethanol, dihydrochloride

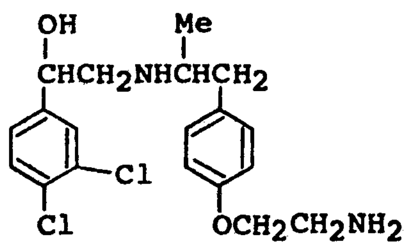

.2HCl

(R,R)-2-[4-[2-[(2-(3,4-Dichlorophenyl)-2-hydroxy-1-oxoethyl)amino]propyl]phenoxy]acetamide (1.0g) was treated with borane-methyl sulphide complex following a similar procedure to that described in Example 3 to give (R,R)-3,4-Dichloro-α-[[[2-[4-[2-[amino]ethyloxy] phenyl]-1-methylethyl]amino]methyl]benzenemethanol, dihydrochloride as a white solid.

$^{1}H$ nmr $\delta$($d_6$-DMSO)

0.9-1.2 (3H,m), 2.0-3.3 (5H,m), 4.0-4.2 (2H,m), 4.5-4.8 (3H,m), 6.5-6.7 (1H, broad), 7.0 (2H,d), 7.3 (2H,d), 7.5-7.8 (3H,m), 8.5 (3H, broad), 9.10 (1H, broad), 9.7 (1H, broad).

EXAMPLE X 5

(R,R)-2-[4-[2-[(2-(3,4-Dichlorophenyl)-2-hydroxy-1-oxoethyl)amino]propyl]phenoxy]acetamide

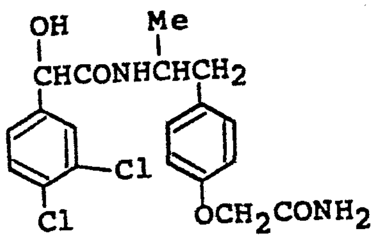

This compound was prepared from (R)-3,4-dichloromandelic acid and (R)-2-[4-[2-amino]propyl]phenoxy] acetamide following the procedure described in Example 1.

$^1$H nmr δ($d_6$-DMSO)

1.05 (3H,d), 2.65 (2H,d), 4.0 (1H,m), 4.4 (2H,s), 4.9 (1H,d), 6.22 (1H,d), 6.8 (2H,d), 7.0 (2H,d), 7.3-7.6 (3H,m + 1H, broad), 7.85 (2H,d).

EXAMPLE X 6

(R,R,R,R)-3-Chloro-α-[[[2-[4-[2-[[2-(4-phenylmethyloxy-phenyl)-2-hydroxyethyl]amino]propyl]phenyl]-1-methyl-ethyl]amino]methyl]benzenemethanol

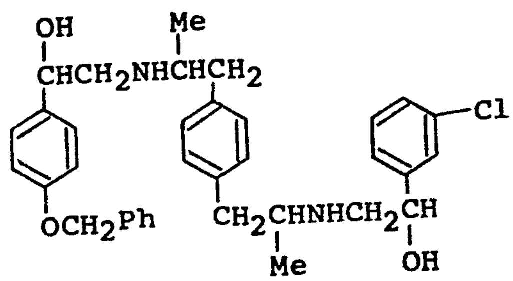

Dicyclohexylcarbodiimide (0.92g) was added to a stirred, ice-cooled solution of (R,R,R)-3-chloro-α-hydroxy-N-[2-[4-[2-aminopropyl]phenyl]-1-methylethyl] benzeneacetamide (1.54g), (R)-4-phenylmethyloxymandelic acid (1.08g) and 1-hydroxybenzotriazole (0.6g) in dry dimethylformamide (80ml) and the solution left to stir at ambient temperature for 18h. The solvent was evaporated, the residue diluted with ethyl acetate, filtered and the filtrate washed successively with sodium carbonate solution, 2N hydrochloric acid, water and dried ($MgSO_4$). Filtration and evaporation of the solvent gave a foam which was chromatographed on silica. Elution with methanol : chloroform (2:98) gave the intermediate bisamide as a foam (2.07g). This was dissolved in dry tetrahydrofuran (60ml) and added dropwise, under nitrogen, to a slurry of lithium aluminium hydride (1.26g) in dry tetrahydrofuran (40ml). The mixture was stirred and heated under reflux for 17h, then cooled and water (1.26g) followed by 2N sodium hydroxide solution (1.26ml) added. The mixture was filtered and evaporated to an oil which was

chromatographed on silica. Elution with methanol : chloroform (6:94) gave (R,R,R,R)-3-chloro-α-[[[2-[4-[2-[[2-(4-phenylmethyloxyphenyl)-2-hydroxyethyl]amino]propyl]phenyl]-1-methylethyl]amino]methyl]benzene- methanol as a white solid (1.25g). Identical material was also prepared by the reaction of (R)-3-chloromandelic acid with (R,R,R)-α-hydroxy-4-phenylmethyloxy-N-[2-[4-[2-aminopropyl]phenyl]-1- methylethyl]benzeneacetamide under the same conditions as described above.

$^1H$ nmr δ($d_6$-DMSO)

0.88 (3H,d), 0.90 (3H,d), 2.39 (2H,dd), 2.62-2.76 (6H,m), 2.78-2.83 (2H,m), 3.0-3.4 (2H, broad, disappears with $D_2O$), 4.51 (1H,t), 4.58 (1H,t), 5.08 (2H,s), 4.75-5.3 (2H, broad, disappears with $D_2O$), 6.94 (2H,d), 7.01 (4H,s), 7.22 (2H,d), 7.25-7.5 (9H,m).

EXAMPLE X 7

(R,R,R)-3-Chloro-α-hydroxy-N-[2-[4-[2-aminopropyl]-phenyl]-1-methylethyl]benzeneacetamide

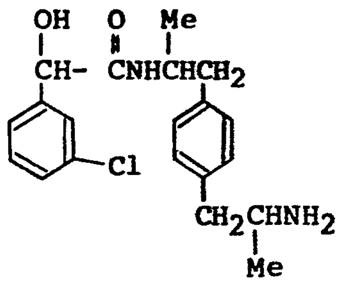

Dicyclohexylcarbodiimide (2.95g) was added to an ice-cooled, stirred solution of (R)-3-chloromandelic acid (2.47g) and 1-hydroxybenzotriazole (1.93g) in dry dimethylformamide (50ml). The mixture was stirred for 1h. A solution of (R,R)-α,α$^1$-dimethyl-1,4-benzene-diethanamine (2.75g) in dry dimethylformamide (50ml) was added. The resulting solution was stirred at ambient temperature for 60h, the solvent evaporated and the residue partitioned between ethyl acetate and 2N sodium hydroxide solution (some insoluble material was removed by filtration). The organic layer was washed with water, dried and evaporated to give a solid which was chromatographed on silica.

Elution with chloroform gave bis acylated material plus some dicyclohexylurea. Change of solvent to methanol-chloroform (8:92) gave (R,R,R)-3-chloro-α-hydroxy-N-[2-[4-[2-aminopropyl]phenyl]-1-methylethyl] benzeneacetamide as a white solid (1.66g), m.p. 149-153$^o$.

$^1$H nmr δ($d_6$-DMSO)
1.0 (3H,d), 1.15 (3H,d), 2.3-3.3 (5H,m), 3.5 (3H, broad, disappears with $D_2O$), 4.2 (1H,m), 4.9 (1H,s), 6.9 (1H,s), 7.05 (4H,s), 7.3 (3H,m), 7.45 (1H,s).

EXAMPLE X 8

(R,R,R)-α-Hydroxy-4-phenylmethyloxy-N-[2-[4-[2-aminopropyl]phenyl]-1-methylethyl]benzeneacetamide

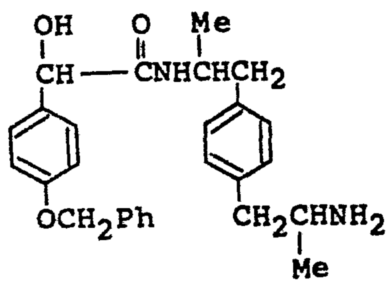

This material (m.p. 126-128°) was prepared in a similar manner to that described in Example X 7 by replacing (R)-3-chloromandelic acid with (R)-4-phenylmethyloxy mandelic acid.

$^1H$ nmr δ($d_6$-DMSO)

1.0 (3H,d), 1.1 (3H,d), 2.3-3.2 (5H,m + 3H, disappears with $D_2O$), 4.2 (1H,m), 4.9 (1H,s), 5.0 (2H,s), 6.8 (2H,d), 7.0 (4H,s), 7.3 (2H,d), 7.45 (5H,s), 7.3-7.4 (1H, broad).

EXAMPLE X 9

3-Chloro-α-(R)-hydroxy-N-[2-[4-[2-(R)-[(3-hydroxy-2-(S)-hydroxypropyl)amino]propyl]phenyl]-1-(R)-methylethyl]benzeneacetamide

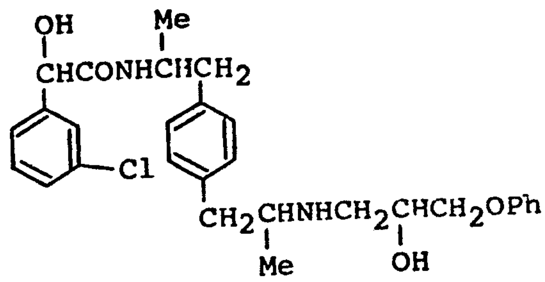

A mixture of (R,R,R)-3-chloro-α-hydroxy-N-[2-[4-[2-aminopropyl]phenyl]-1-methylethyl]benzeneacetamide, Example X 7, (1.31g) and (S)-phenoxymethyloxirane (0.55g) was stirred and heated under reflux in ethanol (30ml) for 24h. The solvent was evaporated to give a clear oil (1.8g), which was chromatographed on silica. Elution with chloroform gave 3-chloro-α-(R)-hydroxy-N-[2-[4-[2-(R)-[bis(3-hydroxy-2-(S)-hydroxypropyl)amino]-propyl]phenyl]-1-(R)-methylethyl]benzeneacetamide. Change of solvent to chloroform-methanol (96:4) gave 3-chloro-α-(R)-hydroxy-N-[2-[4-[2-(R)-[(3-hydroxy-2-(S)-hydroxypropyl)amino]propyl]phenyl]-1-(R)-methylethyl]benzeneacetamide as a clear oil (1.4g).

$^1H$ nmr δ($CDCl_3$)

1.1 (6H,d), 2.3-3.0 (7H,m), 3.3-3.6 (3H, broad), 3.78 (3H,m), 4.0-4.4 (1H), 4.8 (1H,s), 6.6-7.5 (15H,m).

EXAMPLE X 10

4-Amino-3,5-dichloro-α-[[[2-[4-[2-(R)-[[2-(R)-(4-phenylmethyloxyphenyl)-2-hydroxyethyl]amino]propyl]-phenyl]-1-(R)-methylethyl]amino]methyl]benzenemethanol

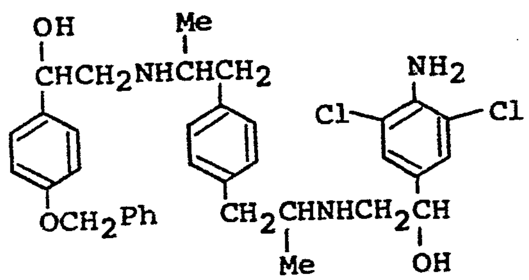

A solution of 4-phenylmethyloxy-α-(R)-hydroxy-N-[2-[4-[2-(R)-[[2-(4-amino-3,5-dichlorophenyl)-2-hydroxyethyl]-amino]propyl]phenyl]-1-(R)-methylethyl]benzeneacetamide, Example X 14, (0.85g), in dry tetrahydrofuran (30ml) was added dropwise, under nitrogen, to a stirred slurry of lithium aluminium hydride (0.71g) in dry tetrahydrofuran (60ml). The mixture was stirred and heated under reflux for 16h, cooled, water (0.71ml) followed by 2N sodium hydroxide solution (0.71ml), added and the resulting mixture filtered. Evaporation of the filtrate gave a colourless oil which was chromatographed on silica. Elution with chloroform-methanol (96:4) gave a mixture of starting material plus 4-phenylmethyloxy-α-(R)-hydroxy-N-[2-[4-[2-(R)-[[2-(4-amino-3,5-dichlorophenyl)ethyl]-amino]propyl]phenyl]-1-(R)-methylethyl]benzeneacetamide. Elution with chloroform-methanol (96:4) gave 4-amino-3,5-dichloro-α-[[[2-[4-[2-(R)-[[2-(R)-(4-phenylmethyloxyphenyl)ethyl]amino]propyl]phenyl]-1-(R)-methylethyl]amino]methyl]benzenemethanol. Elution with

chloroform-methanol (90:10) gave 4-amino-3,5-dichloro-α-[[[2-[4-[2-(R)-[[2-(R)-(4-phenylmethyloxyphenyl)-2-hydroxyethyl]amino]propyl]phenyl]-1-(R)-methylethyl]-amino]methyl]benzenemethanol (0.2g) as a colourless gum.

$^1H$ nmr δ($CDCl_3$)
1.1 (6H,d), 2.3-3 (14H, broad m), 4.3 (2H, broad s), 4.2-4.4 (2H,m), 5.0 (2H,s), 6.9-7.6 (15H,m).

EXAMPLE X 11

<u>4-Phenylmethyloxy-α-(R)-[[[2-[4-[2-(R)-[(3-phenoxy-2-(S)-hydroxypropyl)amino]propyl]phenyl]-1-(R)-methylethyl]amino]methyl]benzenemethanol</u>

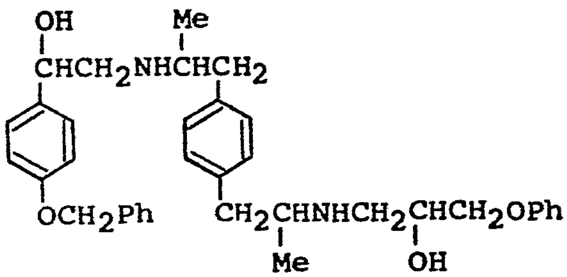

This compound was prepared from the corresponding α-hydroxybenzeneacetamide derivative, Example X 12, following the procedure described in Example 16.

$^1H$ nmr δ($CDCl_3$)

1.15 (3H,d + 3H,d), 2.55-3.2 (14H,m), 3.85 (3H,s), 4.5 (1H,dd), 5.0 (2H,s), 6.7-7.5 (18H,m).

EXAMPLE X 12

4-Phenylmethyloxy-α-(R)-hydroxy-N-[2-[4-[2-(R)-[(3-phenoxy-2-(S)-hydroxypropyl)amino]propyl]phenyl]-1-(R)-methylethyl]benzeneacetamide

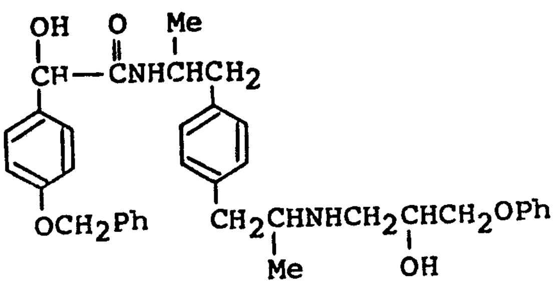

This compound was prepared together with the bis (3-hydroxy-2-(S)-hydroxypropyl) derivative, from the reaction of (S)-phenoxymethyloxirane with (R,R,R)-α-hydroxy-4-phenylmethyloxy-N-[2-[4-[2-aminopropyl]-phenyl-1-methylethyl]benzeneacetamide, Example X 8, following the procedure described in Example X 9.
$^1H$ nmr δ($CDCl_3$)
1.05 (3H,d + 3H,d), 2.4-3.0 (7H,m), 3.1-3.6 (3H, broad), 3.78 (3H,s), 4.25 (1H,m), 4.85 (1H,s), 4.95 (2H,s), 6.6 (1H, broad d), 6.8-7.5 (18H,m).

EXAMPLE X 13

3-Chloro-α-(R)-hydroxy-N-[2-[4-[2-(R)-[[2-(4-amino-3,5-dichlorophenyl)-2-hydroxyethyl]amino]propyl]phenyl]-1-(R)-methylethyl]benzeneacetamide

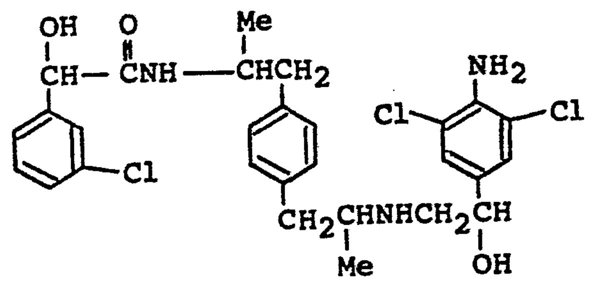

A mixture of (R,R,R)-3-chloro-α-hydroxy-N-[2-[4-[2-aminopropyl]phenyl]-1-methylethyl]benzeneacetamide (1.4g) and 4-amino-3,5-dichlorophenylglyoxal, ethyl hemiacetal (2.05g) was stirred and heated under reflux for 2h in benzene using a Dean and Stark head. The solvent was evaporated, the residue dissolved in methanol, cooled in ice and sodium borohydride (1.0g) added portionwise. After 0.5h the solvent was evaporated, the residue partitioned between ethyl acetate and water and the organic layer separated, dried ($MgSO_4$), filtered and evaporated to give a foam which was chromatographed on silica. Elution with chloroform to chloroform-methanol (98:2) gave 3-chloro-α-(R)-hydroxy-N-[2-[4-[2-(R)-[[2-(4-amino-3,5-dichlorophenyl)-2-hydroxyethyl]amino]propyl]phenyl]-1-(R)-methylethyl]benzeneacetamide as an off-white foam (1.78g).

$^1H$ nmr δ($CDCl_3$)

1.15 (3H,d + 3H,d), 2.0-3.1 (7H,m), 3.2-3.6 (1H,m + 3H, broad), 4.0-4.5 (2H, broad s + 1H,m), 4.8 (1H,s), 6.58 (1H, d, broad), 7.0 (2H,s), 7.02 (4H,s), 7.3 (3H,m), 7.38 (1H,s).

EXAMPLE X 14

4-Phenylmethyloxy-α-(R)-hydroxy-N-[2-[4-[2-(R)-[[2-(4-amino-3,5-dichlorophenyl)-2-hydroxyethyl]amino]propyl]-phenyl]-1-(R)-methylethyl]benzeneacetamide

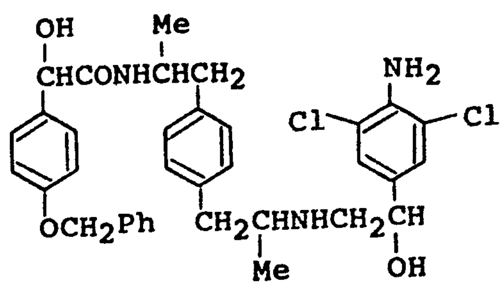

This material was prepared from (R,R,R)-α-hydroxy-4-phenylmethyloxy-N-[2-[4-[2-aminopropyl]phenyl]-1-methylethyl]benzeneacetamide (1.0g) and 4-amino-3,5-dichlorophenylglyoxal, ethylhemiacetal (0.61g) according to the procedure of Example X 13. 4-Phenylmethyloxy-α-(R)-hydroxy-N-[2-[4-[2-(R)-[[2-(4-amino-3.5-dichlorophenyl)-2-hydroxyethyl]amino]propyl]phenyl]-1-(R)-methylethyl]benzeneacetamide, 0.85g, was obtained as a white foam after chromatography on silica.

$^1H$ nmr $\delta(CDCl_3)$

1.1 (6H,d), 2.2-3.1 (7H,m), 3.3-3.9 (3H, broad, m), 4.2 (1H,m), 4.3 (2H,s), 4.85 (1H,s), 5.05 (2H,s), 6.5 (1H,d), 6.8-7.5 (15H,m).

EXAMPLE X 15

4-Phenylmethyloxy-α-(R)-[[[2-[4-[2-(R)-[bis(3-phenoxy-2-(S)-hydroxypropyl)amino]propyl]phenyl]-1-(R)-methylethyl]amino]methyl]benzenemethanol

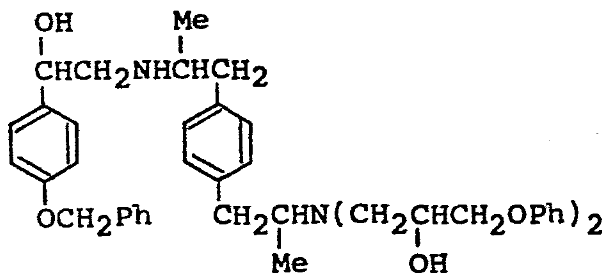

Treatment of 4-phenylmethyloxy-α-(R)-hydroxy-N-[2-[4-[2-(R)-[bis(3-phenoxy-2-(S)-hydroxypropyl)amino]propyl]-phenyl]-1-(R)-methylethyl]benzeneacetamide (see Example X 12) with lithium aluminium hydride according to the procedure described in Example 10 gave 4-phenylmethyloxy-α-(R)-[[[2-[4-[2-(R)-[bis(3-phenoxy-2-(S)-hydroxypropyl)amino]propyl]phenyl]-1-(R)-methylethyl]-amino]methyl]benzenemethanol as a colourless glass.
$^1H$ nmr δ($CDCl_3$)
1.15 (6H,d), 2.5-3.5 (16H,m), 3.7-4.1 (6H,m), 4.5 (1H,dd), 5.0 (2H, s), 6.7-7.6 (23H,m).

EFFECT ON ENERGY EXPENDITURE

The effect of the compounds on the energy expenditure of rats was demonstrated by means of the following procedure:

Male Sprague-Dawley rats each weighing between 170-200g were deprived of food for 16 hours before, and during the experiment. Water was provided ad lib at all times. The compounds were administered orally except where stated in water or water containing dilute hydrochloric acid to 3 or 4 rats. A further 4 rats were dosed orally with water. The rats were placed in boxes through which air was drawn and the oxygen content of the air leaving the boxes was measured. The energy expenditure of the rats was calculated for 3 hours and for 21 hours after dosing from the volume of air leaving the boxes and its oxygen content, following the principles described by J.B. de V. Weir, J. Physiol. (London) 109, 1-9 (1949). The results are expressed as a percentage of the rate of energy expenditure of the rats dosed with water. Results are given in Table 1.

Table 1

| Compound of Example No | Dose mg/kg p.o. | Mean Energy Expenditure (0-3h) | (0-21h) |
|---|---|---|---|
| 1 | 8.7 | 118 | 105 |
| 2 | 10.0 | 141 | 130 |
| 3 | 8.7 | 128 | 97 |
| 4 | 8.1 | 106 | 104 |
| 5 | 5.1 | 122 | 110 |
| 6 | 4.7 | 123 | 115 |
| 7 | 26.7 | 127 | 106 |
| 8 | 54.6 | 97 | 104 |
| 9 | 2.51 | 108 | 110 |
| 10 | 56.6 | 112 | 111 |
| 11 | 11.6 | 115 | 111 |
| 12 | 2.74 | 116 | 104 |
| 13 | 9.29 (ip) | 128 | 105 |
| 14 | 6.0 | 116 | 110 |
| 15 | 9.66 (ip) | 128 | 121 |
| 16 | 11.4 | 117 | 110 |
| 17 | 36.0 | 117 | 111 |
| 18 | 10.6 | 111 | 106 |
| 19 | 9.6 | 113 | 104 |
| 20 | 62.9 | 111 | 108 |
| 21 | 11.0 | 118 | 127 |

## ANTI-HYPERGLYCAEMIC ACTIVITY

Female CFLP mice, weighing approximately 25g, were fasted for 24 hours prior to the study. The compounds under study were administered orally as an aqueous solution to each of 6 mice. Thirty minutes later a blood sample (20µl) was obtained from the tail for the analysis of blood glucose. Immediately after taking this blood sample, glucose (1g/kg body weight) was administered subcutaneously to each mouse. Six mice were given water as a control. Blood samples were then obtained from each mouse at 30 minute intervals for 120 minutes.

Compounds that produced a significant ($p<0.05$) reduction of blood glucose, compared with control mice given water, at any time interval were considered active. The area under the blood glucose curve over the 2-hour period after the administration of the glucose was calculated for each compound and compared with the value for control animals.

| Compounds of Example No | Dose µmol/kg | % Reduction in area under blood glucose curve |
|---|---|---|
| 2 | 25 | 49 |
| 3 | 5 | 10 |
| 4 | 12.5 | 4.2 |
| 5 | 2.5 | 30 |
| 6 | 5 | 48 |

0196849
A

Claims

1. The use of a compound of formula (A):

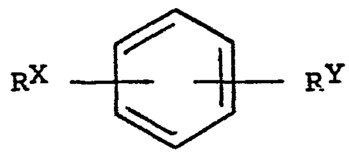 (A)

or a pharmaceutically acceptable salt thereof, wherein $R^X$ is a group of formula (a):

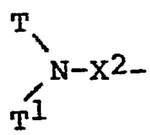 (a)

wherein T represents a group of formula (b):

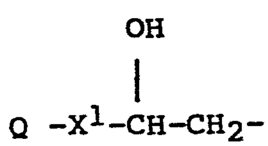 (b)

wherein Q is 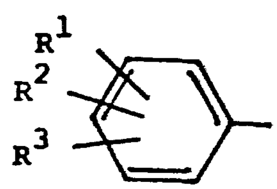 , and wherein

$R^1$ is a hydrogen or halogen atom or a $-CF_3$, $OR^4$, $NHR^4$, $-NHCOR^4$, $-NHCONH_2$, $NHSO_2R^4$, $CH_2OR^4$ or $-CH_2SO_2R^4$ group;

$R^2$ is a hydrogen or halogen atom or an $-OR^4$ group;

$R^3$ is a hydrogen or halogen atom; and $R^4$ is hydrogen, $C_{1-6}$ alkyl or benzyl;

$X^1$ is a bond or $-OCH_2-$;

$X^2$ is a group of formula $-CR^5R^6-CH_2-A-$

wherein $R^5$ and $R^6$ are independently hydrogen or $C_{1-6}$ alkyl; and

A is a bond, $-CH_2-$, or O;

$T^1$ represents a hydrogen atom or a group of formula (b) as defined above in respect of T, such that $T^1$ and T may be the same or different; $R^Y$ represents a group of formula (a), as defined above in respect of $R^X$, such that $R^Y$ and $R^X$ may be the same or different; for the manufacture of a medicament for the treatment of obesity and/or hyperglycaemia.

2. A compound of formula (I):

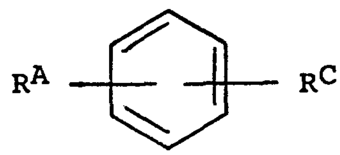

(I)

or a pharmaceutically acceptable salt thereof, wherein $R^A$ is a group of formula (a):

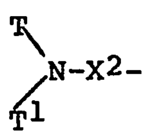

(a)

wherein T represents a group of formula (b):

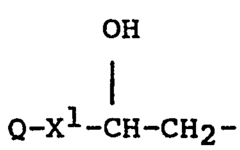

(b)

wherein Q is 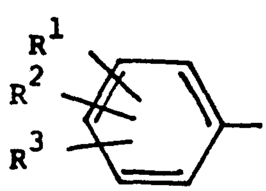 , and wherein

$R^1$ is a hydrogen or halogen atom or a $-CF_3$, $OR^4$, $NHR^4$, $-NHCOR^4$, $-NHCONH_2$, $NHSO_2R^4$, $CH_2OR^4$ or $-CH_2SO_2R^4$ group;

$R^2$ is a hydrogen or halogen atom or an $-OR^4$ group;

$R^3$ is a hydrogen or halogen atom; and $R^4$ is hydrogen, $C_{1-6}$ alkyl or benzyl;

$X^1$ is a bond or $-OCH_2-$;

$X^2$ is a group of formula $-CR^5R^6-CH_2-A-$

wherein $R^5$ and $R^6$ are independently hydrogen or methyl; and

A is a bond, $-CH_2-$, or O;

$T^1$ represents a hydrogen atom or a group of formula (b) as defined above in respect of T, such that $T^1$ and T may be the same or different; $R^C$ represents a group (a) as defined above in respect of $R^A$, such that $R^C$ and $R^A$ may be the same or different; provided that when $R^A$ and $R^C$ each represent a group of formula:

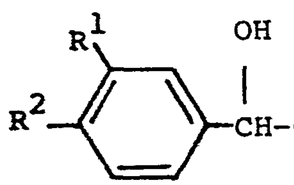

$-CH_2-NH-CH_2-CH_2-A-$ wherein $R^2$ is OH or -O-benzyl; then when A is a bond, $-CH_2-$ or -O, $R^1$ is not $-CH_2OH$ or $NHSO_2-C_{1-4}$ alkyl.

3. A compound as claimed in claim 2 wherein $R^A$ and $R^C$ are para-substituents with respect to each other.

4. A compound as claimed in claim 2 or claim 3, wherein $T^1$ represents hydrogen.

5. A compound as claimed in any one of claims 2 to 4, of formula (II):

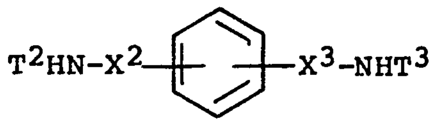

(II)

or a pharmaceutically acceptable salt thereof, wherein $T^2$ and $T^3$ each independently represent a group T as defined in relation to formula (I) in claims 2, and $X^2$ and $X^3$ each independently represent a group of formula $-CR^5R^6-CH_2A$ as defined in relation to formula (I).

6. A compound as claimed in any one of claims 2 to 5, of formula (III):

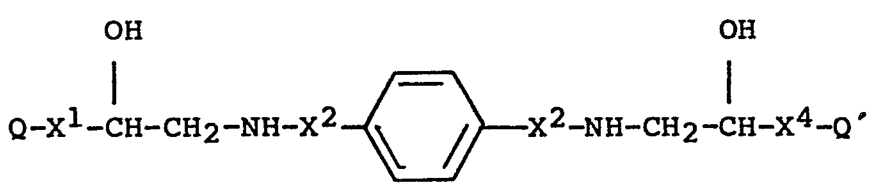

(III)

or a pharmaceutically acceptable salt thereof, wherein $X^2$ is as defined in relation to formula (I) in claim 2, Q and Q′ each independently represent a group

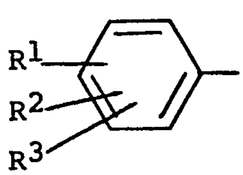

as defined in relation to formula (I) in claim 2; and $X^1$ and $X^4$ each independently represent a bond or $-OCH_2-$

7. A compound as claimed in any one of claims 2 to 6, of formula (IV):

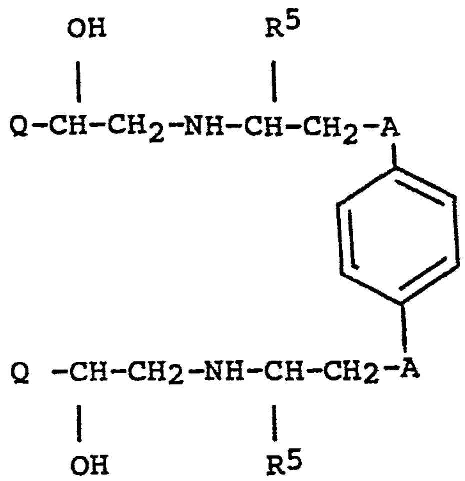

(IV)

or pharmaceutically acceptable salts thereof, wherein Q, A and $R^5$ are as defined in relation to formula (I) in claim 2.

8. A compound as claimed in any one of claims 2 to 6, of formula (V):

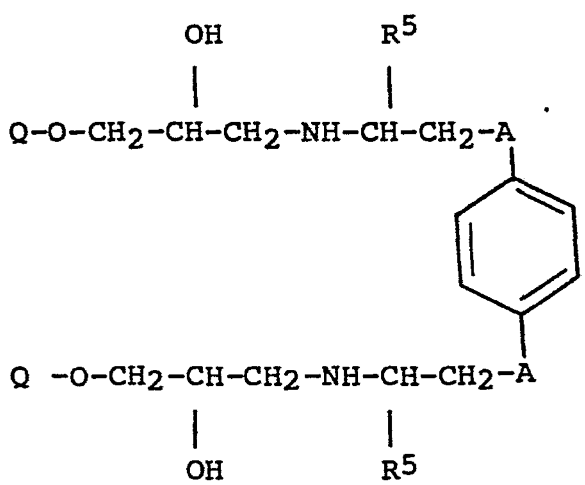

(V)

or a pharmaceutically acceptable salts thereof, wherein Q, A, and $R^5$ are as defined in relation to formula (I) in claim 2.

9. A compound as claimed in any one of claims 2 to 8, wherein Q and Q′ each independently represent phenyl, 3-chlorophenyl, 3,4-dichlorophenyl, 3,5-dichloro-4-amino phenyl or 4-hydroxyphenyl.

10. A compound of formula (A) or (I), selected from the list consisting of:

(R,R,R,R)-α,α'-[1,4-phenylenebis[(1-methyl-2,1-ethanediyl) imino]methylene]bis[benzenemethanol];

(R,R,R,R)-α,α'-[1,4-phenylene bis[(1-methyl-2,1-ethanediyl)imino]methylene]bis[3-chlorobenzenemethanol]

(R,R)-α-α'-[1,4-phenylenedioxybis [(2,1-ethanediyl)-imino] methylene] bis [benzenemethanol];

(R,R)-α,α'-[1,4-phenylenebis [(2,1-ethanediyl) imino]-methylene]bis[benzenemethanol];

(R,R)-α,α1-[1,4-phenylenedioxybis[(2,1-ethanediyl)-imino]methylene]bis[3-chlorobenzene methanol];

(R,R)-α,α'-[1,4-phenylenebis[(2,1-ethanediyl]imino]-methylene]bis[3-chlorobenzene methanol];

α,α'-[1,4-phenylenedioxybis [(1-methyl-2,1-ethanediyl) imino]methylene]bis[(R)-3-chlorobenzenemethanol];

(R,R)-α,α'-[1,2-phenylenebis[(2,1-ethanediyl)imino] methylene] bis [3-chlorobenzenemethanol];

(R,R,R,R)-α,α'-[1,4-phenylenebis [(1-methyl-2,1-ethanediyl)imino] methylene] bis [3,4-dichlorobenzenemethanol];

(R,R,R,R)-1,1'-[1,4-phenylenebis [(1-methyl-2,1-ethanediyl)imino]] bis [3-phenoxypropan-2-ol];

1,1'-[1,4-phenylenebis [((R)-1-methyl-2,1-ethanediyl) imino]]bis [3-phenoxypropan-2-(S)-ol];

(R,R,R)-3,4-dichloro-α-[[[2-[4-[2-[[2-(3-chlorophenyl)-2-hydroxyethyl] amino] ethyloxy] phenyl]-1-methylethyl] amino] methyl] benzenemethanol;

.R,R,R,R)-α,α1-[1,4-phenylenebis [(1-methyl-2,1-ethanediyl)imino]methylene] bis [4-hydroxybenzene-methanol];

α,α1-[1,4-phenylenebis[((R)-1-methyl-2,1-ethanediyl) imino]methylene] bis [(4-amino-3,5-dichloro)benzene-methanol];

(R,R,R,R)-3-chloro-α-[[[2-[4-[2-[[2-(4-hydroxyphenyl)-2-hydroxyethyl]amino]propyl]phenyl]-1-methylethyl] amino]methyl]benzenemethanol;

3-chloro-α-(R)-[[[2-[4-[2-(R)-[(3-phenoxy-2-(S)-hydroxy propyl)amino]propyl]phenyl]-1-(R)-methylethyl]-amino]me thyl]benzenemethanol;

3-chloro-α-(R)-[[[2-[4-[2-(R)-[bis(3-phenoxy-2-(S)-hydr oxypropyl)amino]propyl]phenyl]-1-(R)-methylethyl]-amino ]methyl]benzenemethanol;

4-amino-3,5-dichloro-α-[[[2-[4-[2-(R)-[[2-(R)-(4-hydroxyphenyl)-2-hydroxyethyl]amino]propyl]phenyl]-1-(R)-methylethyl]amino]methyl]benzenemethanol;

4-hydroxy-α-(R)-[[[2-[4-[2-(R)-[(3-phenoxy-2-(S)-hydroxypropyl)amino]propyl]phenyl]-1-(R)-methylethyl]-amino]methyl]benzenemethanol;

4-hydroxy-α-(R)-[[[2-[4-[2-(R)-[bis(3-phenoxy-2-(S)-hydroxypropyl)amino]propyl]phenyl]-1-(R)-methylethyl]-amino]methyl]benzenemethanol; and

4-amino-3,5-dichloro-α-[[[2-[4-[2-(R)-[[2-(R)-(3-chlorophenyl)-2-hydroxyethyl]amino]propyl]phenyl]-1-(R)-methylethyl]amino]methyl]benzenemethanol; or a pharmaceutically acceptable salt thereof.

11. A process for the preparation of a compound of formula (I) as claimed in claim 2 from a compound of formula (VI):

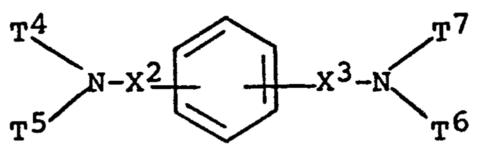

(VI)

wherein $X^2$ and $X^3$ are as defined in relation to formula (II), $T^4$ and $T^7$ each independently represents a hydrogen atom, a nitrogen protecting group or a moiety of formula (b), (c) or (d):

(b) $Q-X^1-CH(OH)-CH_2-$;

(c) $Q-X^1-CH(OH)-CO-$;

(d) $Q-X^1-CO-CH_2-$;

and $T^5$ and $T^6$ each independently represent a hydrogen atom, a nitrogen protecting group or a moiety (b) or $T^4$ together with $T^5$; or $T^6$ together with $T^7$ independently represent a moiety of formula (e)

(e) $Q-X^1-CO-CH=$;

wherein Q and $X^1$ are as defined in relation to formula (I) and providing that either $-X^2-NT^4T^5$ or $-X^3-NT^6T^7$ is not a moiety (a) as defined above, which process comprises carrying out the required number of the following steps:

(i) reducing a moiety (c), (d) or (e) in the compound of formula (VI);

(ii) protecting any -NH- moiety in the compound of formula (VI);

(iii) converting a compound of formula (VI), wherein $T^4$ or $T^7$ represents hydrogen, into another compound of formula (VI) or a compound of formula (I), by reaction with a compound of formula (VIA):

$$Q''-X^1-T^8 \qquad \text{(VIA)}$$

wherein $X^1$ is as defined above, Q'' represents a group Q such that Q and Q'' may be the same or different and $T^8$ represents a moiety (f), (g), (h) or (j):

(f) 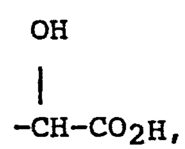

(g) 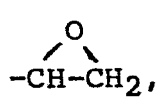

(h) $-CO-CH_2-R^X$,

OH

|

(j) $-CH-CH_2-R^x$,

wherein $R^x$ is a leaving group.

(iv) converting a compound of formula (VI) wherein $-NT^4T^5$ or $-NT^6T^7$ represents $-NH_2$ into another compound of formula (VI), by reaction with a compound of formula (VIA) wherein Q'' and $X^1$ are as defined above and $T^8$ represents a moiety of formula (k)

(k) -CO-CHO

(v) removing any protecting group;

(vi) separating any required product;

(vii) repeating any step (i), (ii), (iii), (iv), (v) or (vi); and if required thereafter forming a pharmaceutically acceptable salt of the compound of formula (I).

12. A compound of formula (VIIB):

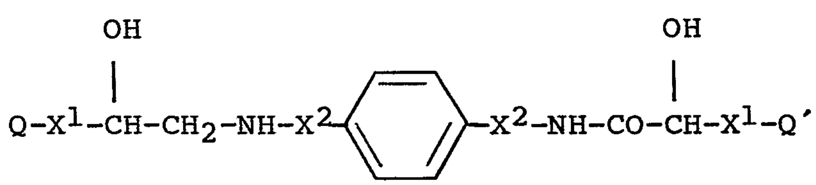 (VIIB)

wherein Q, $X^1$ and $X^2$ are as defined in relation to formula (I) in claim 2.

13. A pharmaceutical composition which comprises an effective non-toxic amount of a compound of formula (I) or (VIIB), or a pharmaceutically acceptable salt thereof, and a pharmaceutically acceptable carrier therefor.

14. A compound of formula (I) or (VIIB), or a pharmaceutically acceptable salt thereof, and a pharmaceutically acceptable carrier therefor, for use as an active therapeutic substance.

15. A method for improving the weight gain and/or improving the feed utilisation efficiency and/or increasing lean body mass and/or decreasing birth mortality rate and increasing post-natal survival rate; of livestock, which method comprises the administration to livestock of a compound of formula (A) or (VIIB) or a veterinarily acceptable salt thereof.

16. A veterinarily acceptable premix formulation comprising a compound of formula (A) or (VIIB) or a veterinarily acceptable salt thereof in association with a veterinarily acceptable carrier.